(19) Europäisches Patentamt
European Patent Office
Office européen des brevets

(11) EP 3 274 689 B1

(12) FASCICULE DE BREVET EUROPEEN

(45) Date de publication et mention
de la délivrance du brevet:
16.02.2022 Bulletin 2022/07

(51) Classification Internationale des Brevets (IPC):
G01N 15/14 (2006.01)        G01N 33/49 (2006.01)
G03H 1/08 (2006.01)         G03H 1/04 (2006.01)

(21) Numéro de dépôt: 16717422.6

(22) Date de dépôt: 23.03.2016

(52) Classification Coopérative des Brevets (CPC):
G01N 15/1429; G01N 15/1463; G03H 1/0443;
G03H 1/0866; G01N 33/49; G01N 2015/1454;
G01N 2015/1488; G03H 2001/0447;
G03H 2001/0883

(86) Numéro de dépôt international:
PCT/FR2016/050643

(87) Numéro de publication internationale:
WO 2016/151248 (29.09.2016 Gazette 2016/39)

(54) **PROCÉDÉ ET DISPOSITIF D'ANALYSE DE PARTICULES**

VERFAHREN UND VORRICHTUNG ZUR ANALYSE VON PARTIKELN

METHOD AND APPARATSU FOR ANALYSING PARTICLES

(84) Etats contractants désignés:
AL AT BE BG CH CY CZ DE DK EE ES FI FR GB
GR HR HU IE IS IT LI LT LU LV MC MK MT NL NO
PL PT RO RS SE SI SK SM TR

(30) Priorité: 24.03.2015 FR 1552443

(43) Date de publication de la demande:
31.01.2018 Bulletin 2018/05

(73) Titulaires:
• Commissariat à l'Energie Atomique et aux
Energies
Alternatives
75015 Paris (FR)
• Horiba ABX SAS
34184 Montpellier Cedex 4 (FR)

(72) Inventeurs:
• ALLIER, Cédric
38000 Grenoble (FR)
• BLANDIN, Pierre
38500 Coublevie (FR)
• ALI CHERIF, Anais
34090 Montpellier (FR)
• HERVE, Lionel
38700 Corenc (FR)

(74) Mandataire: INNOV-GROUP
310, avenue Berthelot
69372 Lyon Cedex 08 (FR)

(56) Documents cités:
EP-A1- 3 137 874          EP-A1- 3 234 550
WO-A1-2015/024020        US-A1- 2009 073 521
US-A1- 2009 290 156      US-A1- 2013 280 752
US-A1- 2014 139 625      US-A1- 2014 327 944

• KEMPER B ET AL: "Application of 3D tracking,
LED illumination and multi-wavelength
techniques for quantitative cell analysis in digital
holographic microscopy", PROCEEDINGS OF
SPIE, S P I E - INTERNATIONAL SOCIETY FOR
OPTICAL ENGINEERING, US, vol. 7184, 26 janvier
2009 (2009-01-26), pages 71840R-1,
XP007913134, ISSN: 0277-786X, DOI:
10.1117/12.808392 ISBN: 978-1-62841-730-2

**Description**

## DOMAINE TECHNIQUE

L'invention se situe dans le domaine du comptage et de l'identification de particules présentes dans un liquide et en particulier un fluide corporel, par exemple du sang.

## ART ANTERIEUR

Les liquides corporels, en particulier le sang, peuvent comprendre des particules, par exemple des cellules, dont il est utile de connaître le nombre et le type.

Dans le sang, par exemple, les analyses de type numération et formule sanguine, ou hémogrammes, sont des examens couramment pratiqués en laboratoire d'analyse. Ce type d'analyse permet de déterminer le nombre et d'identifier les principaux constituants du sang, en particulier des cellules de la lignée des globules rouges, des globules blancs, ou des plaquettes. Ces examens sont couramment pratiqués, à l'aide d'automates performants, mais on recherche des méthodes plus simples, moins onéreuses, permettant d'obtenir des performances comparables.

Un des axes de recherche est l'utilisation de méthodes optiques simples, telle l'imagerie sans lentille. L'observation de particules biologiques par imagerie sans lentille connaît un certain développement depuis la fin des années 2000. Cette technique consiste à intercaler un échantillon entre une source de lumière et un photodétecteur matriciel, ou capteur d'image. L'image recueillie sur le photodétecteur est formée par des interférences entre l'onde incidente, produite par la source de lumière et l'onde diffractée par les particules composant l'échantillon. Cette image est fréquemment désignée par le terme « hologramme ». Ainsi, pour chaque particule, on peut enregistrer au niveau du capteur un motif de diffraction, ou figure de diffraction, qui lui est propre. Appliquée à des échantillons biologiques, cette technique a été décrite dans le document WO2008090330. Il est alors possible d'effectuer une analyse simple de chaque particule, en comparant la figure de diffraction qu'elle engendre avec des figures de diffraction préalablement établies, correspondant à des particules connues. Mais cette méthode peut trouver des limites lorsque la concentration des particules augmente.

En effet, le comptage et l'identification de particules sur la seule base des figures de diffraction détectées par le capteur d'image montre une certaine limite lorsque la concentration de particules dans l'échantillon augmente. En particulier, lorsque l'échantillon est du sang, et que les particules sont des globules rouges, au-delà de 100000 particules par $\mu$l, leur dénombrement n'est plus fiable, comme cela a été reporté dans la publication Seo Sungjyu, « High-throughput lensfree blood analysis on a chip », Anal Chem, 2010 June 1. Il est possible d'appliquer des traitements mathématiques dits de reconstruction holographique numérique afin d'établir une image, dite image complexe, de chaque particule présente dans l'échantillon. Cette méthode consiste à retro-propager l'onde lumineuse dans le plan objet, dans lequel sont situées les particules, ce dernier étant placé à une distance connue de l'imageur. La publication citée ci-dessus montre qu'une telle reconstruction holographique permet d'effectuer un comptage de globules rouges, présents en concentration élevée dans un échantillon. Cette publication montre que, sur l'image complexe reconstruite, les globules blancs, ayant subi un marquage préalable, ont une signature différente de celle des globules rouges.

Le document US2014/0327944 décrit également une méthode de classification de particules, par exemple des particules sanguines, sur la base d'hologrammes, en comparant des hologrammes acquis par un capteur d'image à une bibliothèque d'hologrammes simulés. Mais ce procédé se heurte aux même limites, à savoir une mise en œuvre délicate lorsque la densité des particules est élevée.

Des procédés permettant de reconstruire une image complexe de cellules, en l'occurrence des spermatozoïdes, sont également décrits dans les documents US2012/0218379, ou WO2014/012031. Mais ces méthodes permettent d'estimer des propriétés desdites cellules, ainsi que leur trajectoire, à partir de l'image complexe reconstruite. Il en est de même du document US2009/0290156, qui décrit une classification de particules sur la base d'une image complexe desdites particules, ainsi qu'un suivi de la trajectoire desdites particules. Or, le recours à une image complexe d'un échantillon peut être insuffisant pour identifier une particule.

On recherche un procédé d'identification de particules, et en particulier de cellules sanguines, pouvant s'appliquer à des échantillons dans lesquels la concentration en particules est élevée. Le procédé doit par ailleurs présenter un champ d'observation étendu, et doit être simple à mettre en œuvre, en évitant notamment un marquage préalable de particules. Par ailleurs, le procédé doit permettre de discriminer de façon fiable les particules susceptibles de se trouver dans un fluide corporel, en particulier les cellules des lignées des globules rouges, des globules blancs et des plaquettes.

Le document EP3234550 décrit un procédé d'identification de microorganismes basé sur le recours à des profils formés à partir d'une image acquise selon une configuration défocalisée. Le document US2009139024020 décrit un procédé d'identification de particules basé sur une formation d'un contour tridimensionnel de particules.

Par ailleurs, on recherche une méthode ne nécessitant pas une connaissance précise de la distance entre les particules et le photodétecteur.

## EXPOSE DE L'INVENTION

L'invention répond à ce problème, en proposant un procédé selon la revendication 1 pour identifier une particule présente dans un échantillon, par exemple un échantillon d'un liquide biologique, tel du sang, le procédé comportant les étapes suivantes :

- illumination dudit échantillon à l'aide d'une source de lumière, la source de lumière produisant une onde lumineuse incidente se propageant vers l'échantillon selon un axe de propagation ;
- acquisition, à l'aide d'un photodétecteur matriciel, d'une image de l'échantillon, l'échantillon étant disposé entre ladite source de lumière et ledit photodétecteur, de telle sorte que le photodétecteur matriciel est exposé à une onde lumineuse comprenant des interférences entre l'onde lumineuse incidente et une onde de diffraction produite par chaque particule, aucune optique de grossissement n'étant disposée entre l'échantillon et le photodétecteur matriciel ; le procédé comprend également les étapes suivantes :
- détermination d'une position de ladite particule dans un plan parallèle à un plan selon lequel s'étend le photodétecteur matriciel ;
- application d'un algorithme de reconstruction numérique à ladite image acquise, de façon à estimer au moins une grandeur caractéristique de ladite onde lumineuse à laquelle est exposé le photodétecteur matriciel, à une pluralité de distances de reconstruction de ce dernier ;
- détermination d'un profil, représentant une évolution de ladite grandeur caractéristique en fonction de ladite distance de reconstruction, selon un axe parallèle audit axe de propagation, entre le capteur d'image et la particule, et passant par ladite position ;
- identification de la particule en fonction dudit profil.

Par application d'un algorithme de reconstruction numérique, on entend l'application d'un opérateur de propagation à une image, généralement sous la forme d'un produit de convolution.

La grandeur caractéristique peut être obtenue en estimant, à chaque distance de reconstruction une expression complexe de l'onde lumineuse à laquelle est exposé le photodétecteur matriciel. La grandeur caractéristique peut être déterminée à partir du module de ladite expression complexe, auquel cas elle est représentative de l'amplitude de ladite onde lumineuse à laquelle est exposé le détecteur.

La grandeur caractéristique peut être déterminée à partir de l'argument de ladite expression complexe, auquel cas elle est représentative de la phase de ladite onde lumineuse à laquelle est exposé le photodétecteur matriciel.

Selon un mode de réalisation, le procédé comporte :

- la détermination d'une image complexe, dite image complexe de référence, par application d'un algorithme de reconstruction numérique à l'image acquise par le photodétecteur matriciel ;
- à partir de ladite image complexe de référence, l'estimation d'au moins une grandeur caractéristique de l'onde lumineuse à laquelle est exposé le photodétecteur matriciel, à une pluralité de distances de reconstruction de ce dernier.

Le procédé peut alors comporter :

- l'application d'un opérateur de propagation à l'image complexe de référence, de façon à calculer des images complexes dites secondaires, selon une pluralité de distances du plan de reconstruction ou du plan selon lequel s'étend le photodétecteur matriciel ;
- la détermination d'une grandeur caractéristique à chacune desdites distances, à partir de chaque image complexe secondaire.

L'image complexe de référence peut être une image complexe formée dans un plan de reconstruction, distant du plan de l'échantillon. Il peut également s'agir d'une image complexe formée dans le plan de détection.

L'identification peut être élaborée en comparant l'évolution de la dite grandeur caractéristique à des profils types déterminés au cours d'une phase d'apprentissage.

La position de chaque particule, dans un plan parallèle au plan du photodétecteur matriciel, peut être déterminée à l'aide de l'image acquise par le photodétecteur ou à l'aide de l'expression complexe de l'onde lumineuse à laquelle est exposé le photodétecteur.

La source de lumière est de préférence une source spatialement cohérente, et par exemple une diode électroluminescente, auquel cas un filtre spatial est de préférence disposé entre la source de lumière et l'échantillon. La source de lumière peut être temporellement cohérente, en étant par exemple une diode laser.

Le photodétecteur matriciel comporte une matrice de pixels, aptes à collecter l'onde à laquelle est exposé le photodétecteur. La distance entre les pixels et l'échantillon peut varier entre 50 $\mu$m et 2 cm, et de préférence entre 100 $\mu$m et 5 mm. De préférence l'échantillon n'est pas disposé au contact direct des pixels du photodétecteur.

L'échantillon peut notamment comporter des cellules sanguines. Dans ce cas, les particules peuvent être identifiées parmi les lignées cellulaires des globules blancs, des globules rouges ou plaquettes.

Un autre objet de l'invention est un dispositif selon la revendication 12 pour identifier une particule, la dite particule étant contenue dans un échantillon, le dispositif comprenant :

- une source de lumière agencée pour produire une onde lumineuse incidente, selon un axe de propagation, en direction dudit échantillon ;
- un support, pour maintenir l'échantillon entre ladite source de lumière et un photodétecteur matriciel ;
- le photodétecteur matriciel, agencé pour acquérir une image de l'échantillon, le photodétecteur matriciel étant apte à être exposé à une onde lumineuse, résultant de l'interférence entre ladite onde lumineuse incidente et une onde de diffraction formée par ladite particule, aucune optique de grossissement n'est disposée entre l'échantillon et le photodétecteur matriciel ; le dispositif comporte un processeur, de type calculateur électronique ou microprocesseur, configuré pour mettre en œuvre les opérations suivantes :
- détermination d'une position de ladite particule dans un plan parallèle au plan du photodétecteur matriciel ;
- application d'un algorithme de reconstruction numérique à ladite image acquise, de façon à estimer au moins une grandeur caractéristique de ladite onde lumineuse à laquelle est exposé le photodétecteur matriciel, à une pluralité de distances de reconstruction de ce dernier ;
- détermination d'un profil, représentant l'évolution de ladite grandeur caractéristique en fonction de ladite distance de reconstruction, selon un axe parallèle audit axe de propagation et passant par ladite position, le profil s'étendant entre l'échantillon et le capteur d'image ;
- identification de la particule en fonction dudit profil.

Le processeur peut comporter ou être relié à une mémoire programmable, comprenant une séquence d'instructions permettant la mise en oeuvre des étapes précédemment décrites.

Il peut en particulier être apte à :

- déterminer, à chaque distance de reconstruction, l'expression complexe du rayonnement optique auquel est exposé le détecteur,
- estimer la dite grandeur caractéristique, à chaque distance de reconstruction, en déterminant le module ou l'argument de ladite amplitude complexe.

## FIGURES

La figure 1 représente le dispositif selon un mode de réalisation de l'invention.

La figure 2A représente une image acquise par le photodétecteur matriciel.

La figure 2B représente le profil d'une grandeur caractéristique, dite amplitude complémentaire, de l'onde lumineuse à laquelle est exposé le photodétecteur, en fonction de la distance par rapport au photodétecteur, selon un premier exemple, pour différents types de particules.

La figure 3A représente le profil d'une grandeur caractéristique, dite amplitude complémentaire, de l'onde lumineuse à laquelle est exposé le photodétecteur, en fonction de la distance par rapport au photodétecteur, pour différents globules blancs, selon ce premier exemple.

La figure 3B représente le profil d'une grandeur caractéristique, dite amplitude complémentaire, de l'onde lumineuse à laquelle est exposé le photodétecteur, en fonction de la distance par rapport au photodétecteur, pour différents globules rouges, selon ce premier exemple.

La figure 4A représente une région d'intérêt de l'image acquise par le photodétecteur, centrée sur un agrégat de plaquettes, selon ce premier exemple.

La figure 4B représente le profil d'une grandeur caractéristique, dite amplitude complémentaire, de l'onde lumineuse à laquelle est exposé le photodétecteur, pour différentes plaquettes faisant partie de l'agrégat représenté sur la figure 4A.

La figure 5A représente le profil d'une grandeur caractéristique, dite amplitude complémentaire, de l'onde lumineuse à laquelle est exposé le photodétecteur, en fonction de la distance par rapport au photodétecteur, pour différents types de particules, selon un deuxième exemple.

La figure 5B représente le profil de la phase de l'onde lumineuse à laquelle est exposé le photodétecteur, en fonction de la distance z par rapport au photodétecteur, pour différents types de particules, selon un deuxième exemple.

La figure 6A représente le profil d'une grandeur caractéristique, dite amplitude complémentaire, de l'onde lumineuse à laquelle est exposé le photodétecteur, en fonction de la distance par rapport au photodétecteur, pour différents types de particules, selon un troisième exemple.

La figure 6B représente le profil de la phase de l'onde lumineuse à laquelle est exposé le photodétecteur, en fonction de la distance par rapport au photodétecteur, pour différents types de particules, selon un troisième exemple.

Les figures 7A, 7B et 7C représentent respectivement le profil d'une grandeur composite caractéristique de l'onde lumineuse à laquelle est exposé le photodétecteur, en fonction de la distance, selon les premier, deuxième et troisième exemples.

Les figures 8A, 8B, 8C et 8D représentent respectivement :

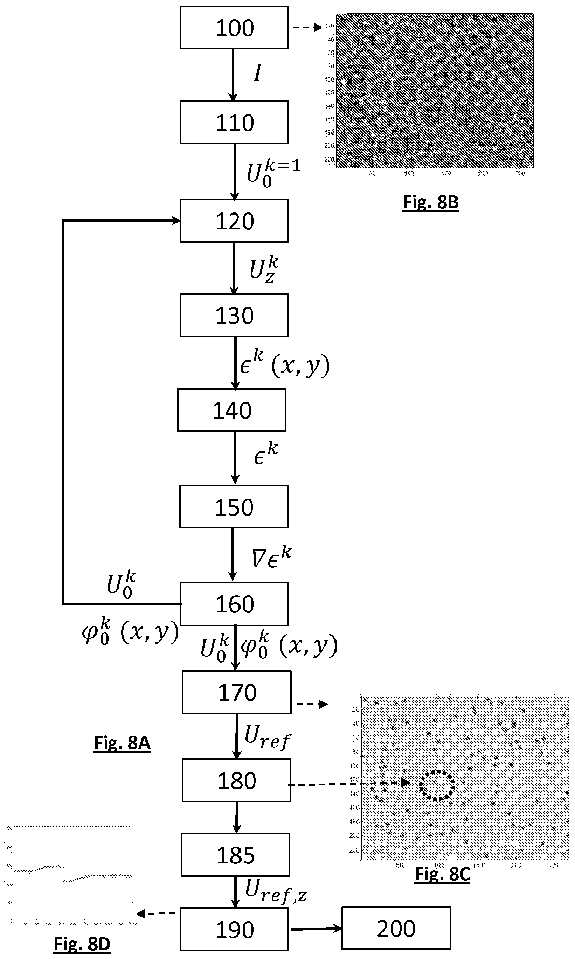

- un procédé permettant le calcul d'une image complexe, dite image complexe de référence, d'un échantillon dans un plan de reconstruction ;
- un hologramme acquis par le photodétecteur matriciel ;
- une représentation d'une image, dite image complexe de référence, reconstruite après plusieurs itérations du procédé représenté sur la figure 8A.
- un profil obtenu sur la base d'images complexes secondaires formées à partir de l'image complexe de référence.

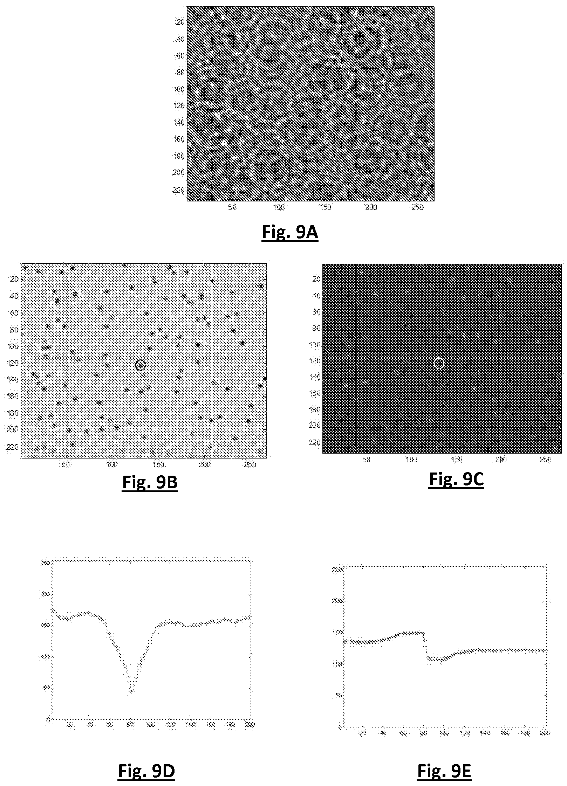

La figure 9A est un hologramme acquis par un capteur d'image, l'échantillon comportant des globules rouges dispersées dans une solution aqueuse. Les figures 9B et 9C représentent respectivement le module et la phase d'une image complexe, dite de référence, cette image complexe étant formée dans un plan de reconstruction. Les figures 9D et 9E sont des profils représentant respectivement une évolution du module et de la phase de l'onde lumineuse auquel le capteur d'image est exposé, le long d'un axe de propagation passant par un globule rouge.

## EXPOSE DE MODES DE REALISATION PARTICULIERS

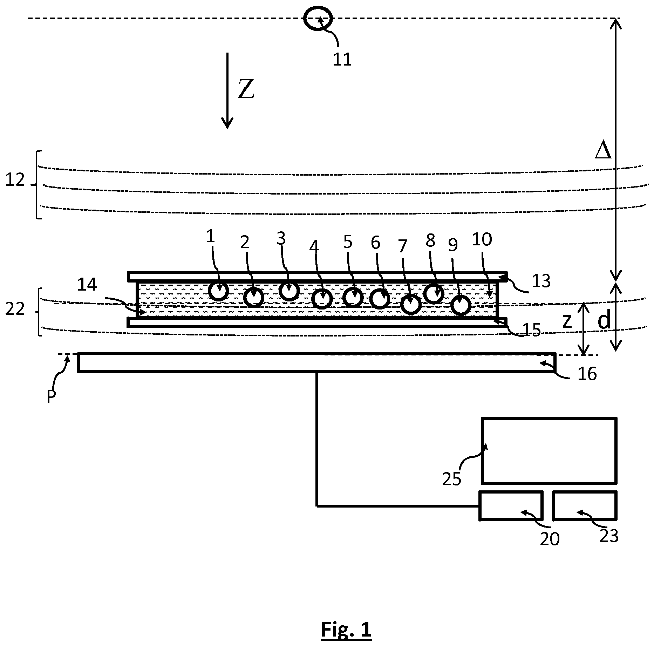

La figure 1 représente un exemple de dispositif objet de l'invention. Une source de lumière 11 est apte à produire une onde lumineuse 12, dite onde lumineuse incidente, en direction d'un échantillon 14, selon un axe de propagation Z. L'échantillon 14 comporte un milieu 10, par exemple un liquide biologique, comportant des particules 1, 2, 3, 4, 5,.., 9, que l'on souhaite identifier parmi des types de particules prédéterminés.

Une particule peut être une cellule. En particulier, lorsque le milieu 10 est du sang, ou une solution comportant du sang, une particule peut être un globule rouge, un globule blanc ou une plaquette.

Une particule peut également être une microbille, organique ou inorganique, par exemple une microbille métallique, une microbille de polymère ou de verre, ce type de microbille étant couramment mis en œuvre dans la réalisation de protocoles biologiques. Une particule peut également être une gouttelette, par exemple une gouttelette lipidique, baignant dans le milieu 10. Il peut également s'agir d'un microorganisme, par exemple une bactérie ou une levure, ou d'un exosome. D'une façon générale, une particule a une taille avantageusement inférieure à 1 mm, voire inférieure à 500 $\mu$m, et de préférence une taille comprise entre 0.5 $\mu$m et 500 $\mu$m. Ainsi, le terme particule désigne à la fois des particules endogènes, initialement présentes dans l'échantillon examiné, et des particules exogènes, ajoutées à cet échantillon avant l'analyse.

Le milieu 10 est le plus fréquemment un milieu liquide, et notamment un liquide corporel, mais il peut également d'agir d'une gélose, ou de l'air, ou le résidu sec d'un liquide.

Le procédé objet de l'invention permet d'identifier chaque particule observée. Par identification, on entend la classification de la particule dans une classe de particules prédéterminée. Il peut s'agir d'une détermination d'une nature d'une particule parmi des natures prédéterminées, ou d'une détermination d'une taille d'une particule parmi des natures prédéterminées.

La distance $\Delta$ entre la source de lumière et l'échantillon est de préférence supérieure à 1 cm. Elle est de préférence comprise entre 2 et 30 cm. De préférence, la source de lumière, vue par l'échantillon, est considérée comme ponctuelle. Cela signifie que son diamètre (ou sa diagonale) est préférentiellement inférieure au dixième, mieux au centième de la distance entre l'échantillon et la source de lumière. Ainsi, la lumière parvient à l'échantillon sous la forme d'ondes planes, ou pouvant être considérées comme telles.

La source de lumière 11 peut être ponctuelle, ou être associée à un diaphragme, ou filtre spatial, non représenté sur la figure 1, de façon à apparaître ponctuelle. L'ouverture du diaphragme est typiquement comprise entre 5 $\mu$m et 1mm, de préférence entre 50 $\mu$m et 500 $\mu$m.

Le diaphragme peut être remplacé par une fibre optique, dont une première extrémité est placée face à une source lumineuse, et dont une deuxième extrémité est placée face à l'échantillon. Dans ce cas, ladite deuxième extrémité peut être assimilée à une source de lumière ponctuelle 11.

L'échantillon 14 est délimité par une enceinte, comportant un fond 15 et un couvercle 13. Les parois latérales de l'enceinte ne sont pas représentées. Dans l'exemple considéré, l'enceinte est une chambre fluidique Neubauer C-chip. La distance entre le fond 15 et le couvercle 13 est de 100 $\mu$m. D'une façon générale, l'épaisseur de l'enceinte, selon l'axe de propagation Z, est inférieure à quelques cm, par exemple inférieure à 1 cm, voire inférieure à 1 mm, par exemple comprise entre 50 $\mu$m et 500 $\mu$m.

La source de lumière 11 peut être temporellement cohérente mais cela n'est pas nécessaire.

Dans ce premier exemple, la source de lumière est une diode Laser émettant à la longueur d'onde de 450 nm. Elle est située à une distance de 15 cm de l'échantillon.

L'échantillon 14 est disposé entre la source de lumière 11 et un photodétecteur matriciel, ou capteur d'image, 16. Ce dernier s'étend de préférence parallèlement, ou sensiblement parallèlement au fond 15 de l'enceinte délimitant l'échantillon.

Le terme sensiblement parallèlement signifie que les deux éléments peuvent ne pas être rigoureusement parallèles, une tolérance angulaire de quelques degrés, inférieure à 10° étant admise.

De préférence, la source de lumière est de faible largeur spectrale, par exemple inférieure à 100 nm, voire 20 nm et encore de préférence inférieure à 5 nm. Le terme largeur spectrale désigne la largeur à mi-hauteur du pic d'émission de la source de lumière.

Le photodétecteur 16 peut être un photodétecteur matriciel, comportant une matrice de pixels, de type CCD ou un CMOS. Les CMOS sont les photodétecteurs préférés car la taille des pixels est plus faible, ce qui permet d'acquérir des images dont la résolution spatiale est plus favorable. Dans cet exemple, le détecteur est un capteur 12 bits APTINA, référence MT9P031. Il s'agit d'un capteur CMOS RGB, dont le pas inter-pixels est de 2,2 $\mu$m. La surface utile du photodétecteur est de $5,7 \times 4,3$ mm$^2$.

Le photodétecteur s'étend selon un plan de détection P, de préférence perpendiculaire à l'axe de propagation Z de l'onde lumineuse incidente 12.

De préférence, le photodétecteur comprend une matrice de pixels, au-dessus de laquelle est disposée une fenêtre de protection transparente. La distance entre la matrice de pixels et la fenêtre de protection est généralement comprise entre quelques dizaines de $\mu$m à 150 ou 200 $\mu$m. Les photodétecteurs dont le pas inter pixel est inférieur à 3 $\mu$m sont préférés, afin d'améliorer la résolution spatiale de l'image.

La distance d entre les particules 1,2,...9 et la matrice de pixels du photodétecteur 16 est, dans cet exemple, égale à 1,5 mm. Mais elle peut fluctuer selon l'épaisseur de la chambre fluidique utilisée. D'une manière générale, et cela quel que soit le mode de réalisation, la distance d entre une particule et les pixels du photodétecteur est préférentiellement comprise entre 50 $\mu$m et 2 cm, de préférence comprise entre 100 $\mu$m et 2 mm.

On remarque l'absence d'optique de grossissement entre le photodétecteur matriciel 16 et l'échantillon 14. Cela n'empêche pas la présence éventuelle de microlentilles de focalisation au niveau de chaque pixel du photodétecteur 16.

Dans ce premier exemple, l'échantillon est du plasma enrichi en globules blancs, obtenu selon un protocole usuel, après sédimentation des globules rouges en présence de Dextran (Sigma Aldrich référence D4876) 6% en solution Alsever, puis récupération du plasma riche globules blancs et en plaquettes. Le plasma obtenu est alors dilué dans un tampon PBS à pH physiologique (acronyme de l'anglais Phosphate Buffer Saline - Tampon Phosphate Salin). La déplétion des globules rouges n'est pas totale et le plasma enrichi obtenu comporte des globules rouges résiduels.

Les particules peuvent être classifiées parmi plusieurs types de particules, et notamment les globules rouges, les globules blancs ou les plaquettes. De préférence, les particules ne subissent aucun marquage préalable.

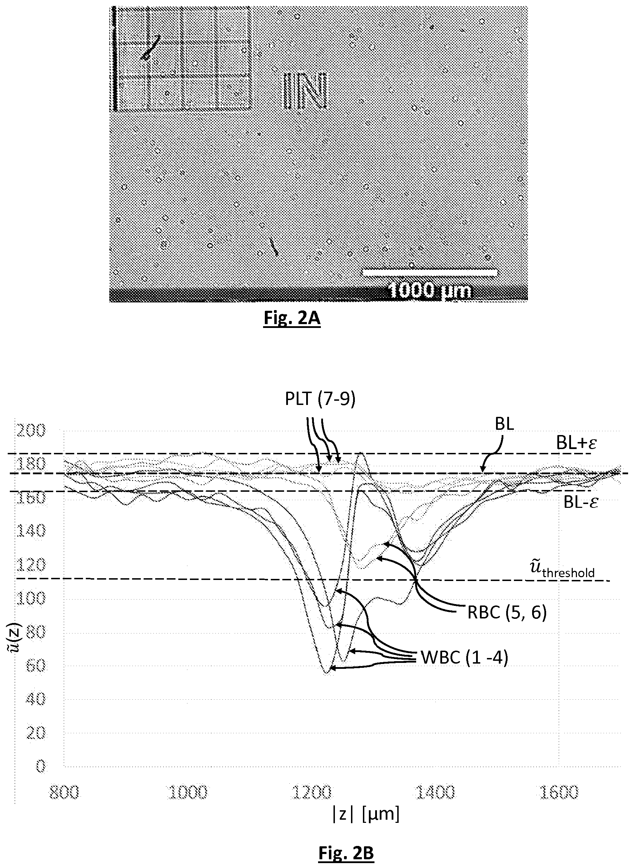

La figure 2A représente une image obtenue par le photodétecteur 16. Cette image représente une figure de diffraction globale, dans laquelle on distingue des figures de diffraction élémentaires, chaque figure de diffraction élémentaire étant respectivement associée aux particules. Chaque figure de diffraction élémentaire comprend une zone centrale en forme de disque, autour de laquelle s'étendent des anneaux concentriques, alternativement sombres et clairs. Une telle figure élémentaire permet la sélection d'une particule à identifier, ainsi que la détermination des coordonnées (x,y) de ladite particule dans le plan de détection P, dites coordonnées radiales. Ces coordonnées sont par exemple le centre de la figure de diffraction élémentaire correspondant à ladite particule.

Chaque figure de diffraction élémentaire est formée par l'interférence entre l'onde lumineuse incidente 12 produite par la source 11, en amont de l'échantillon, et une onde résultant de la diffraction de l'onde incidente par une particule. Ainsi, le photodétecteur 16 est exposé à une onde lumineuse 22 formée par la superposition :

- de l'onde lumineuse 12 émis par la source 11, en amont de l'échantillon 14,

- de l'onde lumineuse diffractée par chacune des particules ou autres éléments diffractants, présents dans l'échantillon 14.

Un processeur 20, par exemple un microprocesseur, reçoit les images du photodétecteur matriciel 16, et effectue une reconstruction de grandeurs caractéristiques de l'onde lumineuse 22 à laquelle est exposé le photodétecteur matriciel, le long de l'axe de propagation Z. Le microprocesseur 20 est relié à une mémoire 23 apte à stocker des instructions pour la mise en œuvre des étapes de calcul décrites dans cette demande. Il peut être relié à un écran 25. La reconstruction est notamment réalisée entre le photodétecteur matriciel et l'échantillon observé.

Le processeur 20 peut être apte à exécuter une séquence d'instructions stockées dans une mémoire, pour la mise en œuvre des étapes du procédé d'identification. Le processeur peut-être un microprocesseur, ou tout autre calculateur électronique apte à traiter les images fournies par le photodétecteur matriciel, pour exécuter une ou plusieurs étapes décrites dans cette description.

L'image *I*, acquise par le photodétecteur matriciel, représentée sur la figure 2A représente la distribution spatiale de l'intensité *I(x,y)*, de l'onde lumineuse 22, *x* et *y* désignant les coordonnées dans le plan P du photodétecteur.

Selon les principes bien connus de la reconstruction holographique numérique, décrits dans la publication Ryle et al, « Digital in-line holography of biological specimens », Proc. Of SPIE Vol.6311 (2006), en effectuant un produit de convolution entre l'intensité *I(x,y)* mesurée par le photodétecteur, et un opérateur de propagation *h(x, y, z)*, il est possible de reconstruire une expression complexe *U(x, y, z)* de l'onde lumineuse 22 en tout point de coordonnées (*x, y, z*) de l'espace, et en particulier dans un plan situé à une distance |z| du photodétecteur.

L'opérateur de propagation *h(x, y, z)* a pour fonction de décrire la propagation de la lumière entre le photodétecteur 16 et un point de coordonnées (x,y,z). Il est alors possible de déterminer l'amplitude *u(x, y, z)* et la phase *φ(x, y, z)* de cette onde lumineuse à cette distance |z|, dite distance de reconstruction, avec :

$$u(x, y, z) = abs\,[U(x, y, z)]\,,$$

$$\varphi(x, y, z) = arg\,[U(x, y, z)]$$

Les opérateurs *abs* et *arg* désignent respectivement le module et l'argument.

L'application de l'opérateur de propagation permet en particulier d'estimer l'expression complexe à une distance |z| du photodétecteur, en amont de ce dernier. On reconstruit alors la valeur complexe de l'onde lumineuse 22 avant que cette dernière n'atteigne le détecteur. On parle alors de rétro-propagation. En attribuant la coordonnée z = 0 au plan de détection P, cette rétro-propagation est mise en œuvre en appliquant un opérateur de propagation *h(x, y, -|z|)*. Les termes en amont et en aval sont à comprendre selon le sens de propagation de l'onde incidente 12.

Si *I(x, y) = I(x, y, z = 0)* correspond à l'intensité du signal mesuré par le photodétecteur, une relation entre l'intensité mesurée *I(x, y)* et l'expression complexe de l'onde lumineuse *U(x, y)*, au niveau du plan de détection P est donnée par : *I(x, y) = |U(x, y)|²*.

L'expression complexe de l'onde lumineuse (22), à une coordonnée (*x, y, z*) est donnée par $U(x, y, z) = \sqrt{I(x, y)} * h(x, y, z)$, le symbole * désignant un produit de convolution. avec :

- z < 0 dans le demi-espace délimité par le plan de détection P, comprenant l'échantillon 14
- z > 0 dans le demi-espace délimité par le plan de détection P et ne comprenant pas l'échantillon 14.

Dans le demi espace délimité par le plan de détection P et comprenant l'échantillon 14, l'expression complexe de l'onde lumineuse peut également s'écrire :

$$U(x, y, z) = \sqrt{I(x, y)} * h(x, y, -|z|)$$

De préférence un pré-traitement mathématique est préalablement appliqué à l'intensité mesurée *I(x, y)* avant la reconstruction holographique. Cela permet d'améliorer la qualité des résultats, notamment en réduisant les artéfacts lors de l'application de l'opérateur de propagation.

Ainsi, on détermine une intensité *Ĩ(x, y)*, dite intensité normalisée, telle que

$$\tilde{I}(x, y) = (I(x, y) - Average\,(I))/Average(I)$$

avec

- *I(x, y)* = intensité mesurée par le photodétecteur à la coordonnée (*x, y*),

- *Average* (*I*) = moyenne de l'intensité mesurée dans une région d'intérêt de l'image *I*, incluant ladite coordonnée (x,y). Cette région d'intérêt peut correspondre à l'image entière formée par le photodétecteur.

Ce pré-traitement s'apparente à une normalisation de l'intensité mesurée par l'intensité de l'onde lumineuse incidente (12), cette dernière étant estimée par l'opérateur *Average* (*I*).

Puis on détermine l'expression complexe de l'onde (22) à partir de l'intensité normalisée $\tilde{I}(x, y)$ selon l'équation

$$U(x, y, z) = \sqrt{\tilde{I}(x, y)} * h(x, y, z)$$ comme préalablement explicité.

La reconstruction numérique peut notamment reposer sur le modèle de diffraction de Fresnel. Dans cet exemple, l'opérateur de propagation est la fonction de Fresnel-Helmholtz, telle que :

$$h(x, y, z) = \frac{1}{j\lambda z} e^{j2\pi\frac{z}{\lambda}} \exp\left(j\pi \frac{x^2 + y^2}{\lambda z}\right).$$

où $\lambda$ désigne la longueur d'onde.

Ainsi, $$U(x, y, z) = \frac{1}{j\lambda z} e^{j2\pi\frac{z}{\lambda}} \iint \sqrt{\tilde{I}(x', y')} \exp\left(j\pi \frac{(x-x')^2 + (y-y'^2)}{\lambda z}\right) dx' dy'$$ où

- x' et *y*' désignent les coordonnées dans le plan du photodétecteur,

- *x* et *y* désignent les coordonnées dans le plan de reconstruction, ce dernier étant situé à une distance |*z*| du photodétecteur,

- z désigne la coordonnée de l'image reconstruite selon l'axe de propagation Z de l'onde lumineuse incidente (12).

A partir des valeurs de l'expression complexe *U*(*x*, *y*, *z*), il est possible d'extraire des grandeurs caractéristiques de l'onde lumineuse 22 résultant de la diffraction, par les particules (1,2..9), du de l'onde lumineuse incidente 12 émise par la source 11. Comme précédemment évoqué, on peut évaluer l'amplitude *u*(*x*, *y*, *z*) ou la phase $\varphi$(*x*, *y*, *z*), mais il est également possible d'évaluer toute fonction de l'amplitude ou de la phase.

On peut, par exemple, évaluer une grandeur caractéristique, dite amplitude complémentaire, $\tilde{u}$(*x*, *y*, *z*) telle que :

$$\tilde{u}(x, y, z) = abs(1 - U(x, y, z))$$

A partir de chaque expression complexe reconstruite U(x,y,z), il est possible de constituer :

- une image $u_z$ de l'amplitude de l'onde 22, selon un plan parallèle au plan du détecteur, à une distance |*z*| de ce dernier, avec $u_z$(*x*, *y*) = *abs* [*U*(*x*, *y*, *z*)],

- une image $\varphi_z$ de la phase de l'onde 22, selon un plan parallèle au plan du détecteur, à une distance |*z*| de ce dernier, avec $\varphi_z$ (*x*, *y*) = *arg* [*U*(*x*,*y*,*z*)],

- une image $\tilde{u}_z$ de l'amplitude complémentaire, telle que précédemment définie, de l'onde 22, selon un plan parallèle au plan du détecteur, à une distance |*z*| de ce dernier, avec $\widetilde{u_z}(x, y, z) = abs[1 - U(x, y, z)]$.

Dans ce premier exemple,

- on reconstruit une image $\widetilde{u_z}$ de l'amplitude complémentaire à une pluralité de coordonnées $z_1...z_M$, selon l'axe de propagation Z, M étant ici égal à 21,

- à partir de chaque image $\widetilde{u_{zm}}$, avec $1 \le m \le M$, on extrait la valeur $\tilde{u}$ ($x_n, y_n, z_m$), ($x_n, y_n$) représentant les coordonnées de la particule n dans un plan parallèle au plan du photodétecteur 16,

- on obtient différentes valeurs de $\tilde{u}$ ($x_n, y_n, z$), avec $z_m < z < z_{m=+1}$ par interpolation entre deux grandeurs $\tilde{u}$ ($x_n, y_n, z_m$) et $\tilde{u}$ ($x_n, y_n, z_{m+1}$) précédemment déterminées.

Les coordonnées $(x_n,y_n)$, dans un plan parallèle au plan du photodétecteur 16, de chaque particule n examinée, sont déterminées soit à l'aide de l'image acquise $I(x, y)$ soit à partir d'une image $\tilde{u}_z$ à une hauteur z de reconstruction donnée.

La figure 2B représente, pour différents types de particules, l'évolution de l'amplitude complémentaire $\tilde{u}(x_n,y_n,z)$, telle que précédemment définie, en fonction de la distance |z| de reconstruction, pour 9 particules différentes :

- particules 1 à 4 : globules blancs désignés par l'acronyme WBC,
- particules 5 et 6 : globules rouges, désignés par l'acronyme RBC,
- particules 7 à 9 : plaquettes, désignées par le lettre PLT.

La distance |z| de reconstruction varie entre $z_{min}$= 1000 et $z_{max}$= 1500 $\mu$m.

Parallèlement à ces opérations, chaque particule (1,...,9) a été observée au microscope, l'observation au microscope servant de mesure de référence, permettant une identification de façon certaine.

On observe que pour l'échantillon étudié dans cet exemple:

- pour les particules 1 à 4, qui sont des globules blancs WBC, la courbe $\tilde{u}(z)$ représentant l'évolution de l'amplitude complémentaire en fonction de la distance de reconstruction présente un minimum inférieur à un seuil d'amplitude $\tilde{u}_{threshold}$, suivi d'une remontée vers la ligne de base BL, cette remontée présentant des oscillations marquées ;
- pour les particules 5 et 6, correspondant à des globules rouges RBC, la courbe $\tilde{u}(z)$ présente un minimum compris entre la ligne de base BL et le seuil d'amplitude $\tilde{u}_{threshold}$, suivi d'une remontée monotone vers la ligne de base BL ;
- pour les particules 7 à 9, correspondant à des plaquettes PLT, la courbe $\tilde{u}(z)$ suit la ligne de base BL et reste confinée entre deux valeurs BL $\pm\ \varepsilon$.

Ainsi, pour chaque particule n détectée, dont la position selon un plan parallèle au plan du détecteur est $(x_n,y_n)$, il est possible d'établir un profil $\tilde{u}(x_n,y_n,z)$ représentant l'évolution de l'amplitude complémentaire à une pluralité de hauteurs de reconstruction z, et d'utiliser ce profil pour effectuer une identification de la particule entre un globule rouge, un globule blanc et une plaquette.

Ce profil peut notamment être comparé à une bibliothèque de profils réalisés, au cours d'une phase d'apprentissage, sur des particules connues. Autrement dit, le profil $\tilde{u}(z)$ représentant l'évolution de l'amplitude complémentaire, selon l'axe de propagation Z (axe des coordonnées z), constitue une signature du type de la particule observée.

Contrairement à l'art antérieur, on ne forme pas une image complexe d'une particule en procédant à une reconstruction holographique à une distance prédéterminée de l'échantillon, mais on reconstruit une caractéristique de l'onde 22, résultant de la diffraction d'une particule avec l'onde incidente 12, le long de la direction propagation de l'onde incidente, à une pluralité de distances du photodétecteur. L'information obtenue est plus riche et permet une classification nette entre différents types de particules.

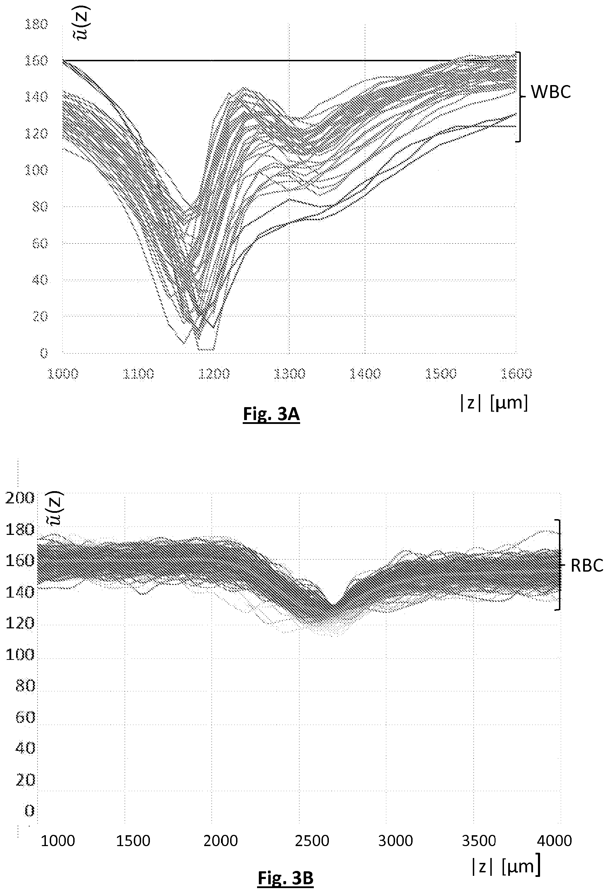

Les figures 3A et 3B représentent des profils d'amplitude complémentaire $\tilde{u}(z)$, de l'onde 22 à laquelle est exposé le détecteur, obtenus respectivement pour 50 globules blancs WBC et 240 globules rouges RBC. On observe une répétabilité suffisante des profils pour permettre une classification robuste des particules sur la base de ce profil. Ces profils ont été obtenus dans des conditions expérimentales analogues à celles de l'exemple précédent.

L'échantillon utilisé pour réaliser les mesures représentées sur la figure 3A est un plasma enrichi similaire à l'échantillon décrit en lien avec les figures 2A et 2B.

L'échantillon utilisé pour réaliser les mesures représentées sur figure 3B comporte du sang total dilué dans un tampon phosphate salin PBS précédemment évoqué, facteur de dilution 1/400.

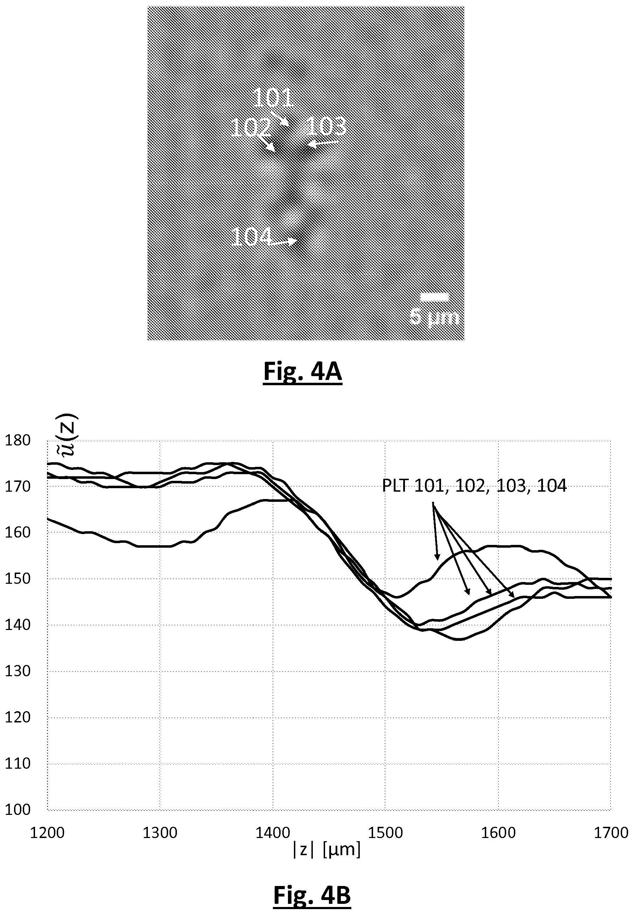

La figure 4B représente des profils d'amplitude complémentaire $\tilde{u}(z)$, selon l'axe Z, obtenus pour 4 plaquettes 101, 102, 103, 104, sur un échantillon de type plasma enrichi tel que précédemment décrit. L'observation au microscope a montré que les plaquettes 101, 102, 103 et 104 étaient agrégées.

La figure 4A représente une région d'intérêt de l'image I acquise par le photodétecteur 16. Elle permet d'identifier les coordonnées $(x_{101},y_{101})$, $(x_{102},y_{102})$, $(x_{103},y_{103})$, $(x_{104},y_{104})$ de chaque plaquette de l'agrégat.

Ces profils ont été obtenus dans des conditions expérimentales analogues à celles du premier exemple. On observe que le profil $\tilde{u}(z)$ est similaire que les plaquettes soient agrégées ou non, et reste confiné autour d'une ligne de base BL, dans un écart BL $\pm\ \varepsilon$. Ainsi, selon le procédé d'identification objet de l'invention, les plaquettes sont correctement identifiées qu'elles soient agrégées ou non.

Dans un deuxième exemple, la source de lumière 11 est une diode électroluminescente blanche couplée à un filtre Omega optical 485-DF-22, centré sur la longueur d'onde $\lambda$=485nm et de largeur à mi-hauteur égale à 22 nm. La distance $\Delta$ entre la source de lumière et le détecteur est égale à 8 cm. L'échantillon est un plasma enrichi tel que précédemment décrit.

Selon cet exemple, on a effectué une reconstruction de l'expression complexe $U(x, y, z)$ de l'onde 22 selon une pluralité de distances z du détecteur, puis on a déterminé, à des coordonnées (x,y) de différentes particules, l'amplitude complémentaire et la phase du rayonnement. On a alors établi des profils $\hat{u}(z)$ et $\varphi(z)$ de l'amplitude complémentaire et de la phase en fonction de z.

De même que dans les exemples précédents, la nature des particules observées a été confirmée par une observation au microscope.

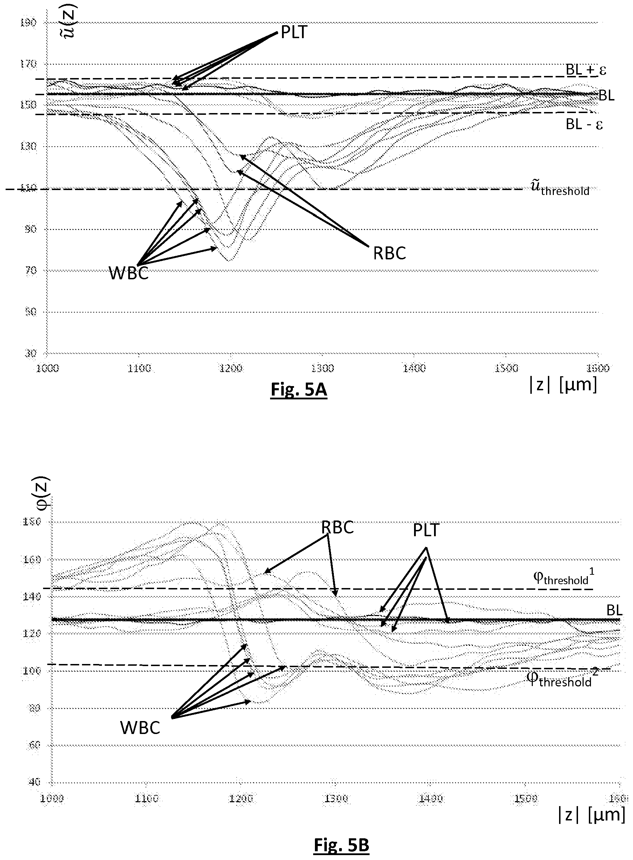

Les figures 5A et 5B représentent respectivement l'amplitude complémentaire $\tilde{u}(x, y, z)$et la phase $\varphi(x, y, z)$ en fonction de la distance z pour différentes particules. Sur la figure 5A, on observe que :

- pour les particules correspondant à des globules blancs WBC, la courbe $\hat{u}(z)$ représentant l'évolution de l'amplitude complémentaire en fonction de la distance de reconstruction présente un minimum marqué inférieur à un seuil d'amplitude $\tilde{u}_{threshold}$, suivi d'une remontée vers la ligne de base BL, cette remontée présentant des oscillations marquées ;
- pour les particules correspondant à des globules rouges RBC, la courbe $\tilde{u}(z)$ présente un minimum compris entre la ligne de base BL et le seuil d'amplitude $\tilde{u}_{threshold}$, puis la courbe suit une remontée monotone vers la ligne de base BL ;
- pour les particules correspondant à des plaquettes PLT, la courbe $\tilde{u}(z)$ suit la ligne de base BL et reste confinée entre deux valeurs BL $\pm \varepsilon$.

Ainsi, pour chaque particule n détectée, dont la position selon un plan parallèle au plan du détecteur est $(x_n, y_n)$, il est possible d'établir un profil $\tilde{u}(x_n, y_n, z)$ représentant l'évolution de l'amplitude complémentaire $\tilde{u}$, telle que précédemment définie, de l'onde 22 à laquelle est exposée le détecteur, à une pluralité de hauteurs de reconstruction z, et d'utiliser ce profil pour effectuer une classification de la particule entre un globule rouge RBC, un globule blanc WBC et une plaquette PLT.

La classification est donc possible avec une source de lumière différente d'une source Laser.

Sur la figure 5B, on observe que :

- pour les particules correspondant à des globules blancs WBC, la courbe $\varphi(z)$ représentant l'évolution de la phase $\varphi$ en fonction de la distance de reconstruction z présente un maximum supérieur à un premier seuil de phase $\varphi_{threshold}^1$, puis un minimum inférieur à un deuxième seuil de phase $\varphi_{threshold}^2$, suivi d'une remontée vers la ligne de base BL ;
- pour les particules correspondant à des globules rouges RBC, la courbe $\varphi(z)$ présente un maximum supérieur audit premier seuil de phase $\varphi_{threshold}^1$ puis un minimum supérieur audit deuxième seuil de phase $\varphi_{threshold}^2$, puis la courbe suit une remontée monotone vers la ligne de base BL ;
- pour les particules correspondant à des plaquettes PLT, la courbe $\varphi(z)$ représentant l'évolution de la phase $\varphi$ en fonction de la distance de reconstruction reste confinée entre les deux valeurs $\varphi_{threshold}^1$ et $\varphi_{threshold}^2$. Les valeurs mesurées demeurent entre lesdits premier et deuxième seuils de phase.

Ainsi, pour chaque particule n détectée, dont la position selon un plan parallèle au plan du détecteur est $(x_n, y_n)$, il est possible d'établir un profil $\varphi(x_n, y_n, z)$ représentant l'évolution de la phase du rayonnement auquel est exposé le détecteur, à une pluralité de hauteurs de reconstruction z, et d'utiliser ce profil pour effectuer une classification de la particule entre un globule rouge, un globule blanc et une plaquette.

Selon un troisième exemple, la source de lumière 11 est une diode électroluminescente blanche couplée à un filtre Omega optical 610-DF-20, centré sur la longueur d'onde $\lambda$=610nm et de largeur à mi-hauteur 20 nm, placée à une distance $\Delta$, égale à 8 cm, de l'échantillon. Le mode opératoire et l'échantillon sont similaires à l'exemple précédent.

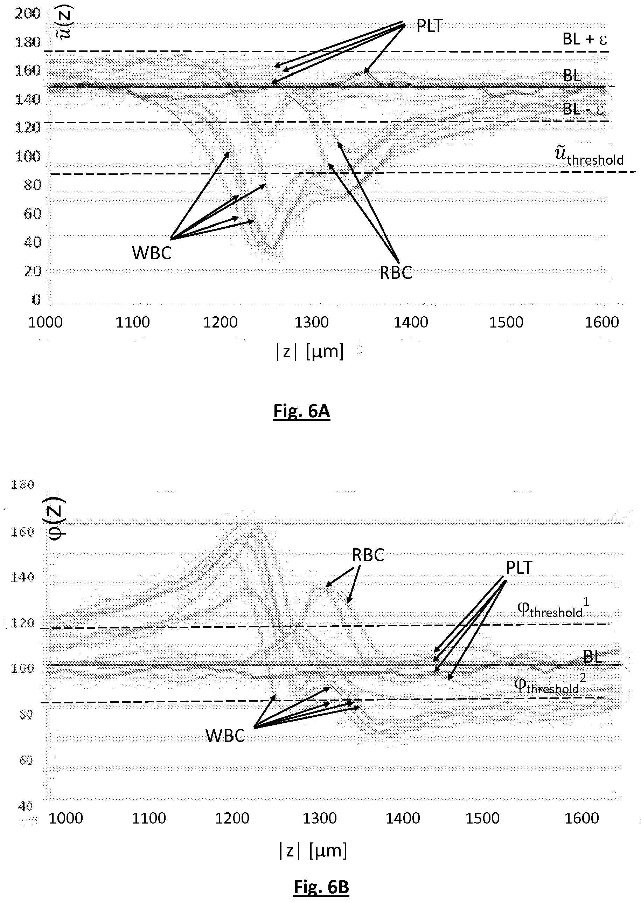

Les figures 6A et 6B représentent respectivement l'amplitude complémentaire $\tilde{u}(x, y, z)$et la phase $\varphi(x, y, z)$ en fonction de la distance z pour différentes particules. Sur la figure 6A, on observe que :

- pour les particules correspondant à des globules blancs WBC, la courbe $\hat{u}(z)$ représentant l'évolution de l'amplitude u en fonction de la distance de reconstruction z présente un minimum marqué inférieur à un seuil d'amplitude complémentaire $\tilde{u}_{threshold}$, suivi d'une remontée vers la ligne de base BL, cette remontée présentant des oscillations marquées ;
- pour les particules correspondant à des globules rouges RBC, la courbe $\tilde{u}(z)$ présente un minimum compris entre la ligne de base BL et le seuil d'amplitude $\tilde{u}_{threshold}$, puis la courbe suit d'une remontée monotone vers la ligne de base BL ;
- pour les particules correspondant à des plaquettes PLT, la courbe $\hat{u}(z)$ suit la ligne de base BL et reste confinée entre deux valeurs BL $\pm \varepsilon$

Ainsi, pour chaque particule n détectée, dont la position selon un plan parallèle au plan du détecteur est $(x_n, y_n)$, il est possible d'établir un profil $\tilde{u}(x_n, y_n, z)$ représentant l'évolution de l'amplitude complémentaire, telle que précédemment définie, du rayonnement auquel est exposé le détecteur, à une pluralité de hauteurs de reconstruction z, et d'utiliser ce profil pour effectuer une classification de la particule entre un globule rouge RBC, un globule blanc WBC et un plaquette PLT.

Sur la figure 6B, on observe que :

- pour les particules correspondant à des globules blancs WBC, la courbe $\varphi(z)$ représentant l'évolution de la phase $\varphi$ en fonction de la distance de reconstruction z présente un maximum supérieur à un premier seuil de phase $\varphi_{threshold}^1$, puis un minimum inférieur à un deuxième seuil de phase $\varphi_{threshold}^2$, suivi d'une remontée vers la ligne de base BL ;
- pour les particules correspondant à des globules rouges RBC, la courbe $\varphi(z)$ présente un maximum supérieur audit premier seuil de phase $\varphi_{threshold}^1$ puis un minimum supérieur audit deuxième seuil de phase $\varphi_{threshold}^2$, puis la courbe suit une remontée monotone vers la ligne de base BL;
- pour les particules correspondant à des plaquettes PLT, la courbe $\varphi(z)$ représentant l'évolution de la phase $\varphi$ en fonction de la distance de reconstruction z reste confinée entre deux valeurs $\varphi_{threshold}^1$, $\varphi_{threshold}^2$ .Les valeurs mesurées demeurent entre lesdits premier et deuxième seuils de phase.

Les deux exemples précédents montrent qu'un profil en amplitude complémentaire $\tilde{u}(z)$ ou en phase $\varphi(z)$ permettent de caractériser les particules.

Il est également possible de constituer un paramètre optique dit composite, noté k, combinant l'amplitude complémentaire et la phase, sous la forme d'un ratio, par exemple

$$k(z) = \frac{\varphi(z)}{\tilde{u}(z)}$$

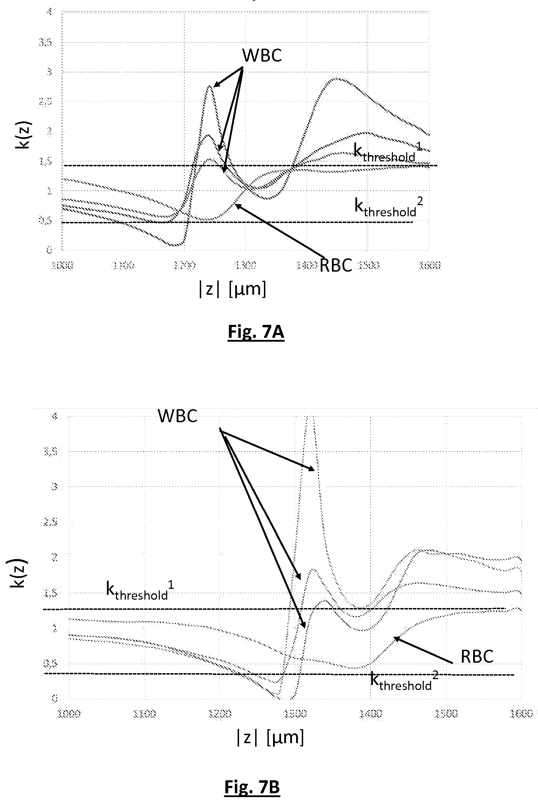

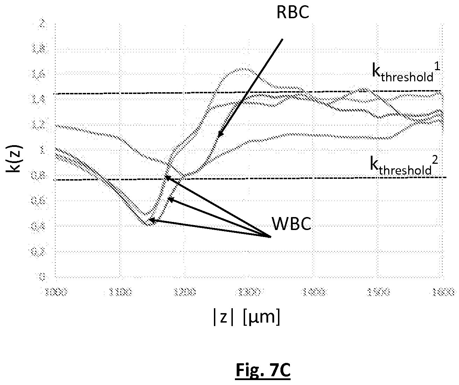

Les figures 7A, 7B et 7C représentent l'évolution dudit paramètre optique composite le long de l'axe de propagation Z, en mettant en œuvre les configurations respectives du premier exemple (source Laser à 405nm), du deuxième exemple (source de lumière de type LED blanche combinée à un filtre centré sur $\lambda$ = 485nm) et du troisième exemple (source de lumière de type LED blanche combinée à un filtre centré sur $\lambda$ = 610nm). Dans chacune des configurations, l'échantillon observé est un plasma enrichi tel que précédemment décrit.

Sur chaque courbe, les particules analysées sont trois globules blancs (WBC) et un globule rouge (RBC).

On observe que, quelle que soit la source, les fluctuations du profil $k(z)$ sont plus importantes pour les globules blancs que pour les globules rouges.

En particulier, il est possible de déterminer un premier seuil composite $k_{threshold}^1$ et un deuxième seuil composite $k_{threshold}^2$, de telle sorte que lorsque le profil $k(z)$ reste inférieur au premier seuil composite $k_{threshold}^1$ et supérieur au deuxième seuil composite $k_{threshold}^2$, la particule analysée est un globule rouge. Lorsque le profil franchit un de ces seuils, la particule examinée est identifiée comme étant un globule blanc.

L'application d'un opérateur de propagation numérique h à une image $I$ acquise, ou hologramme, par un photodétecteur matriciel 16 peut avoir certaines limites, du fait que l'image acquise ne comporte pas d'information relative à la phase. Aussi, préalablement à l'établissement du profil, il est préférable de disposer d'une information relative à la phase de l'onde lumineuse 22 à laquelle est exposé le photodétecteur 16. Cette information relative à la phase peut être obtenue en reconstruisant une image complexe $U_z$ de l'échantillon 14, selon des méthodes décrites dans l'art antérieur, de façon à obtenir une estimation de l'amplitude et de la phase de l'onde lumineuse 22 au niveau du plan P du photodétecteur matriciel 16 ou dans un plan de reconstruction $P_z$ situé à une distance |z| de ce dernier. Les inventeurs ont mis au point un procédé basé sur le calcul d'une image complexe de référence, décrit en lien avec la figure 8A. Ce procédé comprend les étapes suivantes :

- Acquisition d'une image $I$ de l'échantillon 14 par le photodétecteur matriciel 16, cette image formant l'hologramme (étape 100).
- Calcul d'une image complexe, dite de référence, $U_{ref}$ de l'échantillon 14 dans un plan de reconstruction $P_z$ ou dans le plan de détection P, cette image complexe de référence comportant des informations de phase et d'amplitude de l'onde lumineuse 22 à laquelle est exposé le photodétecteur matriciel 16 ; cette étape est effectuée en appliquant l'opérateur de propagation h, précédemment décrit, à l'image acquise $I$ (étapes 110 à 170). Cette image complexe est désignée comme étant une image de référence car elle sert de base à la formation du profil sur la base duquel la particule est caractérisée.

- Sélection d'une position radiale $(x, y)$ d'une particule dans le plan de détection ou dans un plan parallèle à ce dernier (étape 180), soit en utilisant l'image complexe de référence $U_{ref}$, soit l'image $I$ acquise par le photodétecteur 16.

- Application de l'opérateur de propagation *h* à l'image complexe de référence $U_{ref}$ de façon à calculer des images complexes $U_{ref,z}$, dites secondaires, le long de l'axe de propagation Z (étape 185).
- A partir de chaque image complexe secondaire $U_{ref,z}$, estimation d'une grandeur caractéristique de l'onde lumineuse 22, à la position radiale (x,y) de la particule préalablement sélectionnée, et à une pluralité de distances du plan de reconstruction $P_z$ (ou du plan de détection P), puis formation d'un profil représentant une évolution de ladite grandeur caractéristique selon l'axe de propagation Z (étape 190).
- Caractérisation de la particule en fonction de ce profil. Comme précédemment indiqué, cette caractérisation peut être effectuée par une comparaison du profil obtenu avec des profils étalons obtenus lors d'une phase de calibration, à l'aide d'échantillons étalons. (étape 200).

L'algorithme présenté sur la figure 8A est détaillé ci-dessous, les résultats obtenus au cours de certaines étapes étant illustrés sur les figures 8B à 8D. Les étapes 110 à 170 constituent une façon préférée d'obtenir une image complexe de référence, notée $U_{ref}$, cette image représentant une distribution spatiale de l'expression complexe de l'onde 22 dans un plan de reconstruction $P_z$. L'homme du métier comprendra que d'autres algorithmes permettant de reconstruire une telle image complexe, et par exemple les algorithmes cités en lien avec l'art antérieur, sont également envisageables.

Etape 100 : acquisition d'image

Au cours de cette étape, le capteur d'image 16 acquiert une image *I* de l'échantillon 14, et plus précisément de l'onde lumineuse 22 transmise par ce dernier, à laquelle est exposé le capteur d'image. Une telle image, ou hologramme, est représentée sur la figure 8B.

Cette image a été réalisée en utilisant un échantillon 14 comportant des globules rouges baignant dans un tampon salin, l'échantillon étant contenu dans une chambre fluidique d'épaisseur 100 μm disposé à une distance d de 1500 μm d'un capteur CMOS, selon le dispositif précédemment décrit.

Etape 110 : initialisation

Au cours de cette étape, on définit une image initiale $U_0^{k=0}$ de l'échantillon 14, à partir de l'image *I* acquise par le capteur d'image 16. Cette étape est une initialisation de l'algorithme itératif décrit ci-après en lien avec les étapes 120 à 180, l'exposant k désignant le rang de chaque itération. Le module $u_0^{k=0}$ de l'image initiale $U_0^{k=0}$ peut-être obtenu en appliquant l'opérateur racine carrée à l'image acquise *I* par le capteur d'image, auquel cas $u_0^{k=0} = \sqrt{I_0}$. Dans cet exemple, on procède à une normalisation de l'image *I* par un terme représentatif de l'intensité de l'onde lumineuse 12 incidente à l'échantillon 14. Ce dernier peut être, par exemple, la racine carrée d'une moyenne $\bar{I}$ de l'image *I*, auquel cas chaque pixel *I(x, y)* de l'image acquise est divisé par ladite moyenne, de telle sorte que $u_0^{k=0} = \sqrt{\frac{I(x,y)}{\bar{I}}}$

La phase $\varphi_0^{k=0}$ de l'image initiale $U_0^{k=0}$ est soit considérée comme nulle en chaque pixel (*x, y*), soit prédéterminée selon une valeur arbitraire. En effet, l'image initiale $U_0^{k=0}$ résulte directement de l'image *I* acquise par le photodétecteur matriciel 16. Or, cette dernière ne comporte pas d'information relative à la phase de l'onde lumineuse 22 transmise par l'échantillon 14, le capteur d'image 16 n'étant sensible qu'à l'intensité de cette onde lumineuse.

Etape 120 : propagation

Au cours de cette étape, l'image $U_0^{k-1}$ obtenue dans le plan de l'échantillon est propagée dans un plan de reconstruction $P_z$, par l'application d'un opérateur de propagation tel que précédemment décrit, de façon à obtenir une image complexe $U_z^k$, représentative de l'échantillon 14, dans le plan de reconstruction $P_z$. La propagation est réalisée par convolution de l'image $U_0^{k-1}$ par l'opérateur de propagation $h_{-z}$, de telle sorte que : $U_z^k = U_0^{k-1} * h_{-z}$, le symbole * désignant un produit de convolution. L'indice -z représente le fait que la propagation est réalisée dans un sens opposé à l'axe de propagation Z. On parle de rétro-propagation.

Lors de la première itération (*k* =1), $U_0^{k=0}$ est l'image initiale déterminée lors de l'étape 110. Au cours des itérations suivantes, $U_0^{k-1}$ est l'image complexe dans le plan de détection P mise à jour au cours de l'itération précédente.

Le plan de reconstruction $P_z$ est un plan distant du plan de détection $P$, et de préférence parallèle à ce dernier. De préférence, le plan de reconstruction $P_z$ est un plan $P_{14}$ selon lequel s'étend l'échantillon 14. En effet, une image reconstruite dans ce plan permet d'obtenir une résolution spatiale généralement élevée. Il peut également s'agir d'un autre plan, situé une distance non nulle du plan de détection, et de préférence parallèle à ce dernier, par exemple un plan s'étendant entre le photodétecteur matriciel 16 et l'échantillon 14.

Etape 130 : Calcul d'un indicateur en plusieurs pixels

Au cours de cette étape, on calcule une grandeur $\epsilon^k(x, y)$ associée à chaque pixels d'une pluralité de pixels (x,y) de l'image complexe $U_z^k$, et de préférence en chacun de ces pixels. Cette grandeur dépend de la valeur $U_z^k(x, y)$ de l'image $U_z^k$, ou de son module, au pixel $(x, y)$ à laquelle elle est calculée. Elle peut également dépendre d'une dérivée dimensionnelle de l'image en ce pixel, par exemple le module d'une dérivée dimensionnelle de cette image.

Dans cet exemple, la grandeur $\epsilon^k(x, y)$ associée à chaque pixel est un module d'une différence de l'image $U_z^k$, en chaque pixel, et la valeur 1. Une telle grandeur peut être obtenue selon l'expression :

$$\varepsilon^k(x,y) = \sqrt{\left( U_z^k(x,y) - 1 \right)\left( U_z^k(x,y) - 1 \right)^*} = \left| U_z^k(x,y) - 1 \right|$$

Etape 140 : établissement d'un indicateur de bruit associé à l'image $U_z^k$.

Lors de l'étape 130, on a calculé des grandeurs $\epsilon^k(x, y)$ en plusieurs pixels de l'image complexe $U_z^k$. Ces grandeurs peuvent former un vecteur $\mathbf{E}^k$, dont les termes sont les grandeurs $\epsilon^k(x, y)$ associées à chaque pixel (x,y). Dans cette étape, on calcule un indicateur, dit indicateur de bruit, à partir d'une norme du vecteur $\mathbf{E}^k$. D'une façon générale, à une norme est associé un ordre, de telle sorte que la norme $\|\mathbf{x}\|_p$ d'ordre $p$ d'un vecteur $\mathbf{x}$ de dimension $n$ de coordonnées $(x_1, x_2, \ldots x_n)$ est telle que : $\|\mathbf{x}\|_p = \left( \sum_{i=1}^{n} |x_i|^p \right)^{1/p}$, avec $p \geq 0$.

Dans le cas présent, on utilise une norme d'ordre 1, autrement dit $p = 1$. Les inventeurs ont en effet estimé que le recours à une norme d'ordre 1, ou d'ordre inférieur ou égal à 1, est particulièrement adapté à un tel échantillon, comme expliqué ci-après.

Au cours de cette étape, la grandeur $\epsilon^k(x, y)$ calculée à partir de l'image complexe $U_z^k$, à chaque pixel $(x, y)$ de cette dernière, est sommée de façon à constituer un indicateur de bruit $\epsilon^k$ associé à l'image complexe $U_z^k$.

Ainsi,

$$\varepsilon^k = \sum_{(x,y)} \varepsilon^k(x,y)$$

Du fait de l'utilisation d'une norme d'ordre 1, ou d'ordre inférieur ou égal à 1, la valeur de l'indicateur de bruit $\epsilon^k$ diminue lorsque l'image complexe $U_z^k$ est de plus en plus représentative de l'échantillon. En effet, lors des premières itérations, la valeur de la phase $\varphi_0^k(x, y)$, en chaque pixel $(x, y)$ de l'image $U_0^k$ est mal estimée. La propagation de l'image de l'échantillon du plan de détection P vers le plan de reconstruction $P_z$ s'accompagne alors d'un bruit de reconstruction important, comme évoqué en lien avec l'art antérieur. Ce bruit de reconstruction se présente sous la forme de fluctuations apparaissant sur l'image reconstruite. Du fait de ces fluctuations, un indicateur de bruit $\epsilon^k$, tel que précédemment défini est d'autant plus élevé que la contribution du bruit de reconstruction, sur l'image reconstruite, est importante. En effet, les fluctuations dues au bruit de reconstruction tendent à augmenter la valeur de cet indicateur.

Un aspect important de cette étape consiste à déterminer, dans le plan de détection P, des valeurs de phase $\varphi_0^k(x, y)$ de chaque pixel de l'image de l'échantillon $U_0^k$, permettant d'obtenir, lors d'une itération suivante, une image reconstruite $U_z^{k+1}$ dont l'indicateur $\epsilon^{k+1}$ est inférieur à l'indicateur $\epsilon^k$.

Lors de la première itération, comme précédemment expliqué, on ne dispose que d'une information pertinente sur l'intensité de l'onde lumineuse 22, mais non sur sa phase. La première image reconstruite $U_z^{k=1}$ dans le plan de reconstruction $P_z$ est donc affectée d'un bruit de reconstruction important, du fait de l'absence d'information pertinente quant à la phase de l'onde lumineuse 22 dans le plan de détection P. Par conséquent, l'indicateur $\varepsilon^{k=1}$ est élevé. Au cours des itérations suivantes, l'algorithme procède à un ajustement progressif de la phase $\varphi_0^k(x, y)$ dans le plan de détection P, de façon à minimiser progressivement l'indicateur $\varepsilon^k$. L'image $U_0^k$ dans le plan de détection est représentative de l'onde lumineuse 22 dans le plan de détection P, aussi bien du point de vue de son intensité que de sa phase. Les étapes 120 à 160 visent à établir, de façon itérative, la valeur de la phase $\varphi_0^k(x, y)$ de chaque pixel de l'image $U_0^k$, minimisant l'indicateur $\varepsilon^k$, ce dernier étant obtenu sur l'image $U_z^k$ obtenue par propagation de l'image $U_0^{k-1}$ dans le plan de reconstruction $P_z$.

L'algorithme de minimisation peut être un algorithme de descente de gradient, ou de descente de gradient conjugué, ce dernier étant décrit ci-après.

Etape 150 : Ajustement de la valeur de la phase dans le plan de détection.

L'étape 150 vise à déterminer une valeur de la phase $\varphi_0^k(x, y)$ de chaque pixel de l'image complexe $U_0^k$ de façon à minimiser l'indicateur $\varepsilon^{k+1}$ résultant d'une propagation de l'image complexe $U_0^k$ dans le plan de reconstruction $P_z$, au cours de l'itération suivante k+1. Pour cela, un vecteur de phase $\boldsymbol{\varphi_0^k}$ est établi, dont chaque terme est la phase $\varphi_0^k(x, y)$ d'un pixel (x, y) de l'image complexe $U_0^k$. La dimension de ce vecteur est (N$_{pix}$, 1), où N$_{pix}$ désigne le nombre de pixels considérés. Ce vecteur est mis à jour au cours de chaque itération, par l'expression de mise à jour suivante :

$$\varphi_0^k(x, y) = \varphi_0^{k-1}(x, y) + \alpha^k p^k(x, y)$$

où:

- $\alpha^k$ est un entier, désigné par le terme « pas », et représentant une distance ;
- $\boldsymbol{p^k}$ est un vecteur de direction, de dimension (N$_{pix}$, 1), dont chaque terme $p(x, y)$ forme une direction du gradient $\nabla \varepsilon^k$ de l'indicateur $\varepsilon^k$.

Cette équation peut être exprimée sous forme vectorielle, comme suit :

$$\boldsymbol{\varphi_0^k} = \boldsymbol{\varphi_0^{k-1}} + \alpha^k \boldsymbol{p^k}$$

On peut montrer que :

$$\boldsymbol{p^k} = -\boldsymbol{\nabla \varepsilon^k} + \beta^k \boldsymbol{p^{k-1}}$$

où :

- $\nabla \varepsilon^{\boldsymbol{k}}$ est un vecteur de gradient, de dimension (N$_{Pix}$, 1), dont chaque terme représente une variation de l'indicateur $\varepsilon^k$ en fonction de chacun des degrés de liberté des inconnues du problème, c'est-à-dire les termes du vecteur $\boldsymbol{\varphi_0^k}$ ;
- $\boldsymbol{p^{k-1}}$ est un vecteur de direction établi lors de l'itération précédente ;
- $\beta^k$ est facteur d'échelle appliqué au vecteur de direction $\boldsymbol{p^{k-1}}$.

Chaque terme $\nabla \varepsilon^k(x, y)$ du vecteur de gradient $\nabla \varepsilon$, est tel que

$$\nabla \varepsilon^k (r') = \frac{\partial \varepsilon^k}{\partial \varphi_0^k (r')} = \mathrm{Im}\left(U_0^{k^*}\right)(r') . \left(\frac{\left(U_z^k - 1\right)}{\left|U_z^k - 1\right|} * h_z\right)(r')$$

où Im représente l'opérateur partie imaginaire et r' représente une coordonnée (x, y) dans le plan de de détection.

Le facteur d'échelle $\beta^k$ peut être exprimé de telle sorte que :

$$\beta^{(k)} = \frac{\nabla \varepsilon^{(k)} . \nabla \varepsilon^{(k)}}{\nabla \varepsilon^{(k-1)} . \nabla \varepsilon^{(k-1)}}$$

Le pas $\alpha^k$ peut varier selon les itérations, par exemple entre 0.03 au cours des premières itérations et 0.0005 lors des dernières itérations.

L'équation de mise à jour permet d'obtenir un ajustement du vecteur $\boldsymbol{\varphi_0^k}$, ce qui entraîne une mise à jour itérative de la phase $\varphi_0^k(x, y)$ en chaque pixel de l'image complexe $U_0^k$. Cette image complexe $U_0^k$, dans le plan de détection, est alors mise à jour par ces nouvelles valeurs de la phase associée à chaque pixel. Notons que le module de l'image complexe $U_0^k$ n'est pas modifié, ce dernier étant déterminé à partir de l'image acquise par le photodétecteur matriciel 16, de telle sorte que $u_0^k(x, y) = u_0^{k=0}(x, y)$.

Etape 160 : Réitération ou sortie d'algorithme.

Tant qu'un critère de convergence n'est pas atteint, l'étape 160 consiste à réitérer l'algorithme, par une nouvelle itération des étapes 120 à 160, sur la base de l'image complexe $U_0^k$ mise à jour lors de l'étape 150. Le critère de convergence peut être un nombre K prédéterminé d'itérations, ou une valeur minimale du gradient $\nabla \varepsilon^k$ de l'indicateur, ou une différence considérée comme négligeable entre deux vecteurs de phase $\boldsymbol{\varphi_0^{k-1}}$, $\boldsymbol{\varphi_0^k}$ consécutifs. Lorsque le critère de convergence est atteint, on dispose d'une estimation considérée comme correcte d'une image complexe de l'échantillon, dans le plan de détection P ou dans le plan de reconstruction $P_z$.

Etape 170 : Obtention de l'image complexe de référence.

A l'issue de la dernière itération, le procédé peut comprendre une propagation de l'image complexe $U_0^k$ résultant de la dernière itération dans le plan de reconstruction $P_z$, de manière à obtenir une image complexe de référence $U_{ref} = U_z^k$. De façon alternative, l'image complexe de référence $U_{ref}$ est l'image complexe $U_0^k$ résultant de la dernière itération dans le plan de détection P. Lorsque la densité des particules est élevée, cette alternative est cependant moins avantageuse car la résolution spatiale dans le plan de détection P est moins élevée que dans le plan de reconstruction $P_z$, notamment lorsque le plan de reconstruction $P_z$ correspond à un plan $P_{14}$ selon lequel s'étend l'échantillon 14.

La figure 8C représente une image du module $u_z^{k=8}$ de chaque pixel de l'image complexe de référence $U_z^{k=8}$ obtenue dans un plan de reconstruction $P_z$ à l'issue de 8 itérations. La résolution spatiale de cette image permet une bonne identification des coordonnées radiales (x,y) de chaque particule.

Etape 180 : Sélection des coordonnées radiales particule.

Au cours de cette étape, on sélectionne les coordonnées radiales (x, y) d'une particule à partir de l'image de référence $U_{ref} = U_z^{k=30}$, par exemple à partir de l'image de son module $u_{ref} = u_z^{k=30}$ ou de l'image de sa phase $\varphi_{ref} = \varphi_z^{k=30}$. Comme précédemment évoqué, le terme coordonnée radiale désigne une coordonnée dans le plan de détection ou dans le plan de reconstruction. Il est également envisageable d'effectuer cette sélection à partir de

l'hologramme $I_0$ ou à partir de l'image complexe $U_0^k$ obtenue dans le plan de détection suite à la dernière itération. Cependant, lorsque le nombre de particules augmente, il est préférable d'effectuer cette sélection sur l'image formée dans le plan de reconstruction, du fait de sa meilleure résolution spatiale, en particulier lorsque le plan de reconstruction correspond $P_z$ au plan de l'échantillon $P_{14}$. Sur la figure 8C, on a représenté la sélection d'une particule, entourée par un contour en pointillés.

Etape 185 : Application d'un opérateur de propagation

Au cours de cette étape 185, l'image complexe de référence $U_{ref}$ est propagée selon une pluralité de distances de reconstruction, en utilisant un opérateur de propagation h tel que précédemment défini, de façon à disposer d'une pluralité d'images complexes, dites secondaires, $U_{ref,z}$ reconstruites à différentes distances du plan de détection P ou du plan de reconstruction $P_z$. Ainsi, cette étape comprend la détermination d'une pluralité d'images complexes $U_{ref,z}$ telles que :

$$U_{ref,z} = U_{ref} * h_z \text{ avec } z_{min} \leq z \leq z_{max}.$$

Les valeurs $z_{min}$ et $z_{max}$ sont les coordonnées minimales et maximales, selon l'axe Z, selon lesquelles l'image complexe de référence est propagée. De préférence, les images complexes sont reconstruites selon une pluralité de coordonnées z entre l'échantillon 14 et le capteur d'image 16. Les images complexes peuvent être formées de part et d'autre de l'échantillon 14.
Ces images complexes secondaires sont établies par application d'un opérateur de reconstruction holographique $h$ à l'image de référence $U_{ref}$. Or, cette dernière est une image complexe décrivant correctement l'onde lumineuse 22 auquel est exposé le capteur d'image, en particulier au niveau de sa phase, suite aux itérations des étapes 120 à 160. Par conséquent, les images secondaires $U_{ref,z}$ forment un bon descripteur de la propagation de l'onde lumineuse 22 selon l'axe de propagation Z.

Etape 190 : Formation d'un profil

Au cours de cette étape, à partir de chaque image complexe secondaire $U_{ref,z}$, on détermine une grandeur caractéristique, telle que précédemment définie, de l'onde lumineuse 22 de façon à déterminer un profil représentant l'évolution de ladite grandeur caractéristique selon l'axe de propagation Z. La grandeur caractéristique peut être, par exemple le module ou la phase, ou leur combinaison. La figure 8D représente l'évolution de la phase $\varphi(z)$ de l'onde lumineuse 22 le long de l'axe de propagation Z.

Etape 200 : Caractérisation

La particule peut ensuite être caractérisée à partir du profil formé lors de l'étape précédente. De préférence, on dispose d'une base de données de profils étalons formés au cours d'une phase d'apprentissage à l'aide d'échantillons étalons connus. La caractérisation est alors effectuée par une comparaison ou d'une classification du profil formé sur la base des profils étalons.
Ce mode de réalisation a été testé sur des échantillons comportant des globules rouges. Un autre exemple est présenté sur les figures 9A à 9E. Dans ces exemples, l'échantillon comporte des globules rouges dilués dans une solution aqueuse comportant un tampon PBS (Tampon Phosphate Salin) dilué au 1/400. L'échantillon 14 a été placé dans une chambre fluidique 15 d'épaisseur 100 $\mu$m, disposée à une distance de 8 cm de la diode électroluminescente précédemment décrite, dont la bande spectrale est centrée sur 450 nm. L'échantillon est placé à une distance de 1.5 mm du capteur d'image CMOS précédemment décrit. L'ouverture du filtre spatial 18 s'élève à 150 $\mu$m.
La figure 9A représente l'image $I$ acquise par le capteur d'image. Les images du module et de la phase de l'image complexe $U_z^{k=8}$ reconstruite, dans le plan de l'échantillon $P_{10}$, sont respectivement représentées sur les figures 9B et 9C. Ces images ont été obtenues en 8 itérations.

L'image $U_z^{k=8}$ constitue une image de référence $U_{ref}$, à laquelle l'opérateur de propagation $h$ précédemment décrit a été appliqué, de façon à disposer d'une pluralité d'images complexes secondaires $A_{ref,z}$ selon l'axe de propagation Z. Par ailleurs, sur l'image du module ou sur l'image de la phase de l'image de référence, on a identifié un globule rouge, ce dernier étant entouré par des pointillés sur chacune de ces images. Les coordonnées radiales (x,y) de ce globule rouge ont été extraites. A partir des images complexes secondaires $A_{ref,z}$, on a formé un profil u(z) représentatif du module et un profil $\varphi$(z) représentatif de la phase de l'onde lumineuse 22 atteignant le capteur d'image 16. La valeur de chaque point du profil est respectivement obtenue en déterminant le module et la phase d'une image secondaire auxdites coordonnées radiales. Les figures 9D et 9E représentent respectivement le profil du module et de la phase du globule rouge ainsi sélectionné. Le profil a été déterminé entre des coordonnées $z_{min}$ = 1000 $\mu$m et $z_{max}$ = 2000 $\mu$m avec un pas en z de 5 $\mu$m. Le plan de reconstruction est situé à 1380 $\mu$m du plan de détection, ce qui correspond à l'abscisse 76 sur les figures 9D et 9E.

La méthode décrite ne se limite pas au sang et peut être appliquée à d'autres fluides corporels, par exemple l'urine, le liquide céphalorachidien, la moelle osseuse, etc. Par ailleurs, la méthode peut s'appliquer à des liquides non corporels, en particulier pour l'analyse de polluants ou de toxines dans l'eau ou autre solution aqueuse.

La méthode s'applique également à la détection et à l'identification de particules placées dans un milieu non liquide, par exemple une gélose ou le résidu sec d'un liquide corporel, par exemple un frottis sanguin aboutissant à un dépôt étendu de sang sec sur une lame. Dans ce dernier cas, les particules sont isolées les unes des autres par des résidus secs ou de l'air.

Par ailleurs, comme précédemment indiqué, les particules peuvent être endogènes (par exemple des particules sanguines) ou exogènes (microbilles, goutellettes).

Les exemples décrits ci-avant exposent des critères d'identification simples, basés sur l'évolution du profil d'une grandeur caractéristique en fonction de la distance de reconstruction, et de comparaisons à l'aide de seuils pré-établis. La validité des critères est liée au milieu dans lequel sont placées les particules, ainsi qu'au protocole de préparation de l'échantillon. D'autres critères peuvent s'appliquer à des particules ayant subi un protocole de préparation différent. Ainsi, pour un type d'échantillon donné, les critères d'identification peuvent être définis au cours d'une phase d'apprentissage, réalisée sur des échantillons étalons, comportant des particules connues.

De plus, d'autres méthodes de classification, plus complexes, et plus robustes, peuvent être mises en œuvre, sans sortir du cadre de l'invention comme définie dans les revendications.

## Revendications

1. Procédé pour identifier une particule (1, 2,...9, 101...104) contenue dans un échantillon (14), le procédé comportant les étapes suivantes :

   a) illumination dudit échantillon à l'aide d'une source de lumière (11), la source de lumière produisant une onde lumineuse incidente (12) se propageant vers l'échantillon (14) selon un axe de propagation (Z) ;
   b) acquisition, à l'aide d'un photodétecteur matriciel (16), d'une image de l'échantillon, l'échantillon étant disposé entre ladite source de lumière et le photodétecteur matriciel, de telle sorte que le photodétecteur matriciel est exposé à une onde lumineuse (22) comprenant des interférences entre l'onde lumineuse incidente (12) et une onde de diffraction produite par chaque particule, aucune optique de grossissement n'étant disposée entre l'échantillon et le photodétecteur matriciel.; le procédé comprenant également les étapes suivantes :
   c) détermination d'une position de ladite particule dans un plan parallèle (x,y) à un plan (P) selon lequel s'étend le photodétecteur matriciel (16) ;
   d) application d'un algorithme de reconstruction numérique à ladite image acquise, de façon à estimer au moins une grandeur caractéristique (u, $\tilde{u}$, $\varphi$, k) de ladite onde lumineuse (22) à laquelle est exposé le photodétecteur matriciel (16), à une pluralité de distances (|z|) de reconstruction de ce dernier ;
   e) détermination d'un profil, représentant une évolution (u(z), $\tilde{u}$(z), $\varphi$(z), k(z)) de ladite grandeur caractéristique en fonction de ladite distance, selon un axe parallèle audit axe de propagation (Z), entre le capteur d'image et la particule, et passant par ladite position (x,y) ;
   f) identification de la particule en fonction dudit profil.

2. Procédé selon la revendication 1, dans lequel la dite grandeur caractéristique est obtenue en estimant, à chaque distance de reconstruction, une expression complexe (U(x,y,z)) de l'onde lumineuse (22) à laquelle est exposé le photodétecteur matriciel.

3. Procédé selon la revendication 2, dans lequel la grandeur caractéristique est déterminée à partir du module ou de l'argument de ladite expression complexe (U(x,y,z)).

4. Procédé selon la revendication l'une quelconque des revendications précédentes, dans lequel l'identification est élaborée en comparant l'évolution de la dite grandeur caractéristique à des profils types déterminés au cours d'une phase d'apprentissage.

5. Procédé selon l'une quelconque des revendications 1 à 4, dans lequel la position de chaque particule, dans un plan parallèle au plan du photodétecteur matriciel, est déterminée à l'aide de l'image acquise par le photodétecteur ou à l'aide de l'expression complexe ($U(x,y,z)$) de l'onde lumineuse (22) à laquelle est exposé le photodétecteur matriciel.

6. Procédé selon l'une quelconque des revendications 1 à 5, comportant :

- la détermination d'une image complexe de référence ($U_{ref}$), dans un plan de reconstruction ou dans le plan de détection, par application d'un algorithme de reconstruction numérique à l'image acquise par le photodétecteur matriciel (16) ;
- à partir de ladite image complexe de référence, l'estimation d'au moins une grandeur caractéristique (u, $\tilde{u}$, φ, k) de l'onde lumineuse (22) à laquelle est exposé le photodétecteur matriciel (16), à une pluralité de distances (|z|) de reconstruction de ce dernier.

7. Procédé selon la revendication 6, comportant :

- l'application d'un opérateur de propagation (h) à l'image complexe de référence ($U_{ref}$), de façon à calculer des images complexes dites secondaires ($U_{ref,z}$), selon une pluralité de distances du plan de reconstruction ou du plan selon lequel s'étend le photodétecteur matriciel ;
- la détermination d'une grandeur caractéristique à chacune desdites distances, à partir de chaque image complexe secondaire ($U_{ref,z}$).

8. Procédé selon l'une quelconque des revendications précédentes, dans lequel la source de lumière est une source spatialement cohérente.

9. Procédé selon l'une quelconque des revendications précédentes, dans lequel la source de lumière est une diode électroluminescente ou une diode laser.

10. Procédé selon l'une quelconque des revendications précédentes, dans lequel l'échantillon comporte des cellules sanguines.

11. Procédé selon l'une quelconque des revendications précédentes, les particules étant identifiées parmi les lignées cellulaires des globules blancs, des globules rouges ou des plaquettes.

12. Dispositif pour identifier une particule, la dite particule étant contenue dans un échantillon (14), le dispositif comprenant :

- une source de lumière (11) agencée pour produire une onde lumineuse incidente (12), selon un axe de propagation (Z), en direction dudit échantillon (14) ;
- un support, pour maintenir l'échantillon (14) entre ladite source de lumière (11) et un photodétecteur matriciel (16) ;
- le photodétecteur matriciel (16) étant agencé pour acquérir une image de l'échantillon, en étant exposé à une onde lumineuse (22), résultant de l'interférence entre ladite onde lumineuse incidente (12) et une onde de diffraction formée par ladite particule, aucune optique de grossissement n'est disposée entre l'échantillon et le photodétecteur matriciel; le dispositif en outre comprenant un processeur (20), notamment du type microprocesseur, configuré pour mettre en œuvre les étapes suivantes :
- détermination d'une position de ladite particule dans un plan parallèle ($x, y$) à un plan (P) selon lequel s'étend le photodétecteur matriciel (16) ;
- application d'un algorithme de reconstruction numérique à ladite image acquise, de façon à estimer au moins une grandeur caractéristique (u, $\tilde{u}$, φ, k) de ladite onde lumineuse (22) à laquelle est exposé le photodétecteur matriciel (16), à une pluralité de distances (|z|) de reconstruction de ce dernier ;
- détermination d'un profil, représentant l'évolution ($u(z)$, $\tilde{u}(z)$, $φ(z)$, $k(z)$) de ladite grandeur caractéristique en fonction de ladite distance de reconstruction, selon un axe parallèle audit axe de propagation et passant par ladite position ($x,y$), le profil s'étendant entre l'échantillon et le capteur d'image ;
- identification de la particule en fonction dudit profil.

13. Dispositif selon la revendication 12, dans lequel le processeur est apte à :

- déterminer, à chaque distance de reconstruction, l'expression complexe (*U(x, y, z)*) du rayonnement optique auquel est exposé le détecteur ;
- estimer la dite grandeur caractéristique, à chaque distance de reconstruction, en déterminant le module ou l'argument de ladite amplitude complexe (*U(x, y, z)*).

## Patentansprüche

1. Verfahren zur Identifikation eines Partikels (1, 2,...9, 101...104), das in einer Probe (14) enthalten ist, wobei das Verfahren die folgenden Schritte aufweist:

   a) Beleuchten der Probe mithilfe einer Lichtquelle (11), wobei die Lichtquelle eine einfallende Lichtwelle (12) erzeugt, die sich gemäß einer Ausbreitungsachse (Z) zur Probe (14) hin ausbreitet;
   b) Erfassen eines Bilds der Probe mithilfe eines Matrixphotodetektors (16), wobei die Probe so zwischen der Lichtquelle und dem Matrixphotodetektor angeordnet ist, dass der Matrixphotodetektor einer Lichtwelle (22) ausgesetzt ist, die Interferenzen zwischen der einfallenden Lichtwelle (12) und einer Beugungswelle, die von jedem Partikel erzeugt wird, umfasst, wobei keine Vergrößerungsoptik zwischen der Probe und dem Matrixphotodetektor angeordnet ist;
   wobei das Verfahren ebenfalls die folgenden Schritte umfasst:
   c) Bestimmen einer Position des Partikels in einer Ebene parallel (x, y) zu einer Ebene (P), gemäß welcher sich der Matrixphotodetektor (16) erstreckt;
   d) Anwenden eines Algorithmus zur digitalen Rekonstruktion auf das erfasste Bild, um mindestens eine charakteristische Größe ($u$, $\tilde{u}$, $\phi$, $k$) der Lichtwelle (22) zu schätzen, welcher der Matrixphotodetektor (16) ausgesetzt ist, bei einer Mehrzahl von Rekonstruktionsabständen (|z|) des letzteren;
   e) Bestimmen eines Profils, das eine Fortentwicklung ($u(z)$, $\tilde{u}(z)$, $\phi(z)$, $k(z)$) der charakteristischen Größe in Abhängigkeit von dem Abstand gemäß einer Achse darstellt, die parallel zur Ausbreitungsachse (Z), zwischen dem Bildsensor und dem Partikel, und durch die Position (x, y) verläuft;
   f) Identifizieren des Partikels in Abhängigkeit von dem Profil.

2. Verfahren nach Anspruch 1, wobei die charakteristische Größe durch Schätzung, bei jedem Rekonstruktionsabstand, eines komplexen Ausdrucks (U(x, y, z)) der Lichtwelle (22), welcher der Matrixphotodetektor ausgesetzt ist, erhalten wird.

3. Verfahren nach Anspruch 2, wobei die charakteristische Größe anhand des Moduls oder Arguments des komplexen Ausdrucks (U(x, y, z)) bestimmt wird.

4. Verfahren nach Anspruch einem der vorhergehenden Ansprüche, wobei die Identifikation durch Vergleich der Fortentwicklung der charakteristischen Größe mit typischen Profilen erstellt wird, die im Verlauf einer Lernphase bestimmt werden.

5. Verfahren nach einem der Ansprüche 1 bis 4, wobei die Position jedes Partikels in einer Ebene parallel zur Ebene des Matrixphotodetektors mithilfe des Bilds, das vom Photodetektor erfasst wurde, oder mithilfe des komplexen Ausdrucks (U(x, y, z)) der Lichtwelle (22), welcher der Matrixphotodetektor ausgesetzt ist, bestimmt wird.

6. Verfahren nach einem der Ansprüche 1 bis 5, aufweisend:

   - das Bestimmen eines komplexen Referenzbilds ($U_{ref}$) in einer Rekonstruktionsebene oder in der Detektionsebene durch Anwendung eines Algorithmus zur digitalen Rekonstruktion auf das Bild, das vom Matrixphotodetektor (16) erfasst wurde;
   - das Schätzen, anhand des komplexen Referenzbilds, mindestens einer charakteristischen Größe ($u$, $\ddot{u}$, $\phi$, $k$) der Lichtwelle (22), welcher der Matrixphotodetektor (16) ausgesetzt ist, bei einer Mehrzahl von Rekonstruktionsabständen (|z|) des letzteren.

7. Verfahren nach Anspruch 6, aufweisend:

   - das Anwenden eines Ausbreitungsoperators (h) auf das komplexe Referenzbild (Uref), um komplexe Sekundärbilder ($U_{ref,z}$) zu berechnen, gemäß einer Mehrzahl von Abständen von der Rekonstruktionsebene oder der Ebene, gemäß welcher sich der Matrixphotodetektor erstreckt;

- das Bestimmen einer charakteristischen Größe bei jedem der Abstände anhand jedes komplexen Sekundärbilds ($_{Uref,z}$).

8. Verfahren nach einem der vorhergehenden Ansprüche, wobei die Lichtquelle eine räumlich kohärente Quelle ist.

9. Verfahren nach einem der vorhergehenden Ansprüche, wobei die Lichtquelle eine Leuchtdiode oder Laserdiode ist.

10. Verfahren nach einem der vorhergehenden Ansprüche, wobei die Probe Blutzellen aufweist.

11. Verfahren nach einem der vorhergehenden Ansprüche wobei die Partikel aus den Zelllinien der weißen Blutkörperchen, der roten Blutkörperchen oder Blutplättchen identifiziert werden.

12. Vorrichtung zur Identifikation eines Partikels, wobei das Partikel in einer Probe (14) enthalten ist, wobei die Vorrichtung Folgendes umfasst:

   - eine Lichtquelle (11), die angeordnet ist, eine gemäß einer Ausbreitungsachse (Z) in Richtung der Probe (14) einfallende Lichtwelle (12) zu erzeugen;
   - einen Träger, um die Probe (14) zwischen der Lichtquelle (11) und einem Matrixphotodetektor (16) zu halten;
   - wobei der Matrixphotodetektor (16) angeordnet ist, ein Bild der Probe zu erfassen, wobei er einer Lichtwelle (22) ausgesetzt ist, die aus der Interferenz zwischen der einfallenden Lichtwelle (12) und einer Beugungswelle, die von dem Partikel erzeugt wird, resultiert, wobei keine Vergrößerungsoptik zwischen der Probe und dem Matrixphotodetektor angeordnet ist;
   wobei die Vorrichtung ferner einen Prozessor (20) umfasst, insbesondere vom Typ Mikroprozessor, der dazu ausgebildet ist, die folgenden Schritte umzusetzen:
   - Bestimmen einer Position des Partikels in einer Ebene parallel (x, y) zu einer Ebene (P), gemäß welcher sich der Matrixphotodetektor (16) erstreckt;
   - Anwenden eines Algorithmus zur digitalen Rekonstruktion auf das erfasste Bild, um mindestens eine charakteristische Größe (u, ü, $\phi$, k) der Lichtwelle (22) zu schätzen, welcher der Matrixphotodetektor (16) ausgesetzt ist, bei einer Mehrzahl von Rekonstruktionsabständen (|z|) des letzteren;
   - Bestimmen eines Profils, das die Fortentwicklung (u(z), ü(z), $\phi$(z), k(z)) der charakteristischen Größe in Abhängigkeit von dem Rekonstruktionsabstand gemäß einer Achse darstellt, die parallel zur Ausbreitungsachse und durch die Position (x, y) verläuft, wobei sich das Profil zwischen der Probe und dem Bildsensor erstreckt;
   - Identifizieren des Partikels in Abhängigkeit von dem Profil.

13. Vorrichtung nach Anspruch 12, wobei der Prozessor zu Folgendem geeignet ist:

   - Bestimmen, bei jedem Rekonstruktionsabstand, des komplexen Ausdrucks (U(x, y, z)) der optischen Strahlung, welcher der Detektor ausgesetzt ist;
   - Schätzen der charakteristischen Größe bei jedem Rekonstruktionsabstand durch Bestimmung des Moduls oder Arguments der komplexen Amplitude (U(x, y, z)).

**Claims**

1. Method for identifying a particle (1, 2,...9, 101... 104) contained in a sample (14), the method comprising the following steps:

   a) illuminating said sample using a light source (11), the light source producing an incident light wave (12) propagating towards the sample (14) along a propagation axis (Z);
   b) acquiring, using a matrix-array photodetector (16), an image of the sample, the sample being placed between said light source and the matrix-array photodetector in such a way that the matrix-array photodetector is exposed to a light wave (22) comprising interference between the incident light wave (12) and a diffraction wave produced by each particle, no magnifying optics being placed between the sample and the matrix-array photodetector; the method also comprising the following steps:
   c) determining a position of said particle in a plane (x, y) parallel to a plane (P) in which the matrix-array photodetector (16) lies;
   d) applying a digital reconstruction algorithm to said acquired image, so as to estimate at least one characteristic quantity (u, $\tilde{u}$, $\varphi$, k) of said light wave (22) to which the matrix-array photodetector (16) is exposed, at a plurality of reconstruction distances (|z|) from the latter;

e) determining a profile, representing a variation (u(z), $\tilde{u}$(z), φ(z), k(z)) in said characteristic quantity as a function of said distance, along an axis parallel to said propagation axis (Z), between the image sensor and the particle, and passing through said position (x, y);

f) identifying the particle depending on said profile.

2. Method according to Claim 1, wherein said characteristic quantity is obtained by estimating, at each reconstruction distance, a complex expression (U(x,y,z)) of the light wave (22) to which the matrix-array photodetector is exposed.

3. Method according to Claim 2, wherein the characteristic quantity is determined from the modulus or from the argument of said complex expression (U(x,y,z)).

4. Method according to claim any one of the preceding claims, wherein the identification is achieved by comparing the variation in said characteristic quantity to reference profiles determined in a learning phase.

5. Method according to any one of Claims 1 to 4, wherein the position of each particle, in a plane parallel to the plane of the matrix-array photodetector, is determined using the image acquired by the photodetector or using the complex expression (U(x,y,z)) of the light wave (22) to which the matrix-array photodetector is exposed.

6. Method according to any one of Claims 1 to 5, comprising:

- determining a reference complex image ($U_{ref}$), in a reconstruction plane or in the detection plane, by applying a digital reconstruction algorithm to the image acquired by the matrix-array photodetector (16);
- from said reference complex image, estimating at least one characteristic quantity (u, $\tilde{u}$, φ, k) of the light wave (22) to which the matrix-array photodetector (16) is exposed, at a plurality of reconstruction distances (|z|) from the latter.

7. Method according to Claim 6, comprising:

- applying a propagation operator (h) to the reference complex image ($U_{ref}$), so as to calculate secondary complex images ($U_{ref,z}$) for a plurality of distances from the reconstruction plane or from the plane in which the matrix-array photodetector lies;
- determining a characteristic quantity at each of said distances, from each secondary complex image ($U_{ref,z}$).

8. Method according to any one of the preceding claims, wherein the light source is a spatially coherent source.

9. Method according to any one of the preceding claims, wherein the light source is a light-emitting diode or a laser diode.

10. Method according to any one of the preceding claims, wherein the sample comprises blood cells.

11. Method according to any one of the preceding claims, the particles being identified among cell lines of white blood cells, red blood cells or platelets.

12. Device for identifying a particle, said particle being contained in a sample (14), the device comprising:

- a light source (11) that is arranged to produce an incident light wave (12), along a propagation axis (Z), in the direction of said sample (14);
- a holder, for holding the sample (14) between said light source (11) and a matrix-array photodetector (16);
- the matrix-array photodetector (16) being arranged to acquire an image of the sample, on being exposed to a light wave (22) resulting from interference between said incident light wave (12) and a diffraction wave formed by said particle, no magnifying optics being placed between the sample and the matrix-array photodetector; the device further comprising a processor (20), notably of the microprocessor type, configured to implement the following steps:
- determining a position of said particle in a plane (x,y) parallel to a plane (P) in which the matrix-array photodetector (16) lies;
- applying a digital reconstruction algorithm to said acquired image, so as to estimate at least one characteristic quantity (u, $\tilde{u}$, φ, k) of said light wave (22) to which the matrix-array photodetector (16) is exposed, at a plurality of reconstruction distances (|z|) from the latter;

- determining a profile, representing the variation ($u(z)$, $\tilde{u}(z)$, $\varphi(z)$, $k(z)$) in said characteristic quantity as a function of said reconstruction distance, along an axis parallel to said propagation axis and passing through said position (x,y), the profile extending between the sample and the image sensor;
- identifying the particle depending on said profile.

13. Device according to Claim 12, wherein the processor is configured to:

- determine, at each reconstruction distance, the complex expression ($U(x,y,z)$) of the optical radiation to which the detector is exposed;
- estimate said characteristic quantity, at each reconstruction distance, by determining the modulus or the argument of said complex amplitude ($U(x, y, z)$).

**Fig. 1**

**Fig. 2A**

**Fig. 2B**

**Fig. 3A**

**Fig. 3B**

**Fig. 4A**

**Fig. 4B**

**Fig. 5A**

**Fig. 5B**

**Fig. 6A**

**Fig. 6B**

**Fig. 7A**

**Fig. 7B**

**Fig. 7C**

100

$I$

110

$U_0^{k=1}$

120

$U_z^k$

130

$\epsilon^k(x,y)$

140

$\epsilon^k$

150

$\nabla\epsilon^k$

$U_0^k$

160

$\varphi_0^k(x,y)$

$U_0^k | \varphi_0^k(x,y)$

**Fig. 8A**

170

$U_{ref}$

180

185

$U_{ref,z}$

190 → 200

**Fig. 8B**

**Fig. 8C**

**Fig. 8D**

**Fig. 9A**

**Fig. 9B**

**Fig. 9C**

**Fig. 9D**

**Fig. 9E**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008090330 A
- US 20140327944 A
- US 20120218379 A
- WO 2014012031 A
- US 20090290156 A
- EP 3234550 A
- US 2009139024020 A

**Littérature non-brevet citée dans la description**

- **SEO SUNGJYU.** High-throughput lensfree blood analysis on a chip. *Anal Chem,* 01 Juin 2010
- **RYLE et al.** Digital in-line holography of biological specimens. *Proc. Of SPIE,* 2006, vol. 6311